# EUROPEAN PATENT APPLICATION

(11) **EP 4 796 131 A1**
(43) Date of publication of application: **26.08.2026**
(21) Application number: 25159913.0
(22) Date of filing: 25.02.2025
(51) Int. Cl.: A61L 2/02, C02F 1/48, F16L 58/00

(54) **PREVENTION OF MICROBIOLOGICAL GROWTH IN FLUID-PROCESSING EQUIPMENT**

(71) Applicant: EPFF Electrical Pipe For Fluid transport AB, 736 32 Kungsör (SE)
(72) Inventor: ANDERSSON, Thomas, 736 32 KUNGSÖR (SE)
(74) Representative: Öberg & Sjöberg AB

(57) **Abstract**

A fluid-processing equipment (10) for processing a fluid susceptible to microbiological growth is proposed. The equipment (10) comprises: a first element (12), an actuator (20), and one or more first magnets (16). The first element (12) is configured to be exposed to the fluid. The actuator (20) is arranged to cause a relative motion between the first element (12) and the first magnets (16). Each of the first magnets (16) is arranged to form eddy currents in the first element (12) that inhibits microbiological growth on the first element (12) at the relative motion.

## Description

### Technical field

The technology proposed herein relates to the prevention of microbiological growth fluid-processing equipment.

### Background

Microbiological growth in fluid-processing equipment is a recognized problem. This is particularly the case for food processing equipment, such as in diary processing. The feed that is processed have a high nutrient content and the rate of microbiological growth in the equipment is typically high. Extensive biofilms can form in a few hours. Frequent cleaning is typically required to prevent a drop in quality of the produced product or in the performance of the equipment. For example, microbiological growth may contaminate the produced product, or it may obstruct the flow of the fluid within the equipment. Typically, production must be interrupted for cleaning and chemicals are commonly used, which contributes to a decreased efficiency and increased production costs.

Microbiological growth may also be a problem in other types of fluid-processing equipment, for example used in hydraulic applications. This is particularly the case if biodegradable fluids are used, or if the fluids contain water.

Microbiological growth may be a particular problem in equipment that that cannot be easily disassembled for manual cleaning. Equipment having rotating components, such as centrifuge pumps, extruders, rotary-type positive-displacement pumps, and mixers are typically difficult to disassemble are typically difficult to clean. Similarly, equipment having reciprocating elements, such as piston pumps, plunger pumps, and rotary pistons pumps are typically difficult to clean. Chemicals are typically used when cleaning such equipment, and the equipment must be operated in the cleaning process. The required downtime may be costly Thus, there is a need for reducing microbiological growth in such equipment.

### Object of the proposed technology

The proposed technology aims at preventing or reducing microbiological growth, such as the formation of biofilms, in fluid-processing equipment having a rotating element or reciprocating element. A particular object of the proposed technology is to prevent or reduce the formation of biofilms in such equipment employed in food processing.

### Summary

In a first aspect of the proposed technology, a fluid-processing equipment, or apparatus, for processing a fluid susceptible to microbiological growth is suggested. The equipment comprises: a first element, an actuator, or a plurality of actuators, and one or more first magnets. The first element is configured to be exposed to the fluid, and the actuator is, or the actuators are, arranged to cause, or generate, a relative motion between the first element and the first magnets. Each of the first magnets is, or the first magnets are, arranged to form eddy currents in the first element at the relative motion. More specifically, each of the first magnets is, or the first magnets are, arranged to form eddy currents in the first element that inhibit, or reduce, microbiological growth, or the formation of a biofilm, on the first element at the relative motion.

In a second aspect of the proposed technology, a method for preventing microbiological growth in a fluid-processing equipment, or apparatus is suggested. The equipment comprises: a first element and an actuator, or a plurality of actuators. The first element is configured to be exposed to a fluid, and the actuator is, or the actuators are, arranged to cause, or generate, a relative motion between the first element. The method comprises: arranging, or providing, one or more first magnets to form eddy currents in the first element at the relative motion. The method further comprises: operating the actuator, or the actuators, to cause, or generate, the relative motion. More specifically, the method comprises: arranging, or providing, one or more first magnets to form eddy currents in the first element that inhibits, or reduces, microbiological growth, or the formation of a biofilm, on the first element at the relative motion. It is understood that the fluid is susceptible to microbiological growth.

It is understood that that each of the first magnets forms, is arranged to form, or generate, a magnetic field.
It is further understood that each of the first magnets is arranged to subject the first element, or at least a portion of the first element, to the magnetic field. It is further understood that each of the first magnets may be arranged to subject the complete first element to the magnetic field.

It is understood that the first magnets may be spaced apart from the first element. For each first magnet, it is understood that first magnet has two poles, or a north pole and a south pole, and the first magnet may be positioned and/or oriented with one of the poles facing the first element, or more specifically with the north pole or the south pole facing the first element. It is further understood that there may be a single first magnet. It is further understood that the first element may be composed of several smaller parts that are joined together. It is further understood that the first element is exposed to the fluid in operation of the equipment.

It is understood that there may be a single actuator. It is further understood that the actuator is, or the actuators are, operationally connected, or operationally coupled, to the first element and the first magnets. It is further understood that the actuator is, or the actuators are, arranged to operate the equipment.

It is understood that the fluid-processing equipment may be arranged to, or configured to, change inherent properties of the fluid when operating. For example, it may comprise a centrifuge separator configured to form a lighter phase and a heavier phase of the fluid, or a homogenizer configured to change the size of particles in the fluid.

It is understood that the fluid-processing equipment may be arranged to, or configured to, change static or dynamic properties of the fluid. For example, it may comprise a pump configured to increase a pressure of the fluid, a pump configured to increase a flow rate of the fluid, or a valve configured to regulate a flow of the fluid.

It is specified that the fluid is susceptible to microbiological growth. For example, this may be bacterial growth. The fluid may contain water. The fluid may be a liquid, such as raw milk or raw juice. It is understood that the fluid may be, or contain, a liquid, such as water. It is further understood that the fluid may contain solid particles. The fluid may be a paste, or a minced or pureed product.

The equipment may further comprise: a second element. The one or more first magnets may be stationary, or have a fixed position, relative to second element. The first magnets may be located at the second element. More specifically, the first magnets may be supported by, or attached to, the second element. Alternatively, the first magnets may be spaced apart from the second element. For example, the equipment may comprise a first magnet support that supports the first magnets relative to the second element. The first magnet support may space the first magnets apart from the second element.

The first magnets may be partly or fully embedded in the second element. The second element may have an inside and an outside. More specifically, the second element may have an inner side and an outer side. The first element may be located on, or at, the inside, or the inner side, of the second element. It is understood that the second element may enclose the first element. The first magnets may be located on the inside, or at the inner side, of the second element. Alternatively, the first magnets may be located on the outside of, or at the outer side, of the second element.

The first magnets may be isolated from the fluid. Worded differently, the first magnets may be prevented from contacting the fluid. For example, the equipment may comprise a first cover that covers the first magnets and isolates the first magnets from the fluid. The first cover may be formed by the second element. Worded differently, the second element may be configured to prevent the fluid from contacting the first magnets. More specifically, the first magnets may be fully embedded in the second element. It is understood that the second element then covers the first magnets and prevents the fluid from contacting the first magnets.

It is specified that the actuator is, or the actuators are, arranged to cause a relative motion between the first element and the first magnets. More specifically, the actuator, or actuators, may be arranged to cause a relative motion between the first element and the second element. It is then understood that the actuator is, or the actuators are, operationally connected, or operationally coupled, to the first element and the second element.

It is understood that the second element may be composed of several smaller parts that are joined together.

The relative motion may be a rotating relative motion. Worded differently, the relative motion may be a relative rotation. The rotating relative motion may be relative to, or around, a rotational axis.

The equipment may have a rotary support that operatively connects, or operatively couples, the first element and the first magnets, or more specifically the first element and the second element. It is understood that the rotary support rotationally supports the first element relative to the first magnets, or vice versa, or more specifically that the rotary support rotationally supports the first element relative to the second element, or vice versa.

The relative motion may be a linear relative motion, or linear displacement. Worded differently, the relative motion may be a relative translation. The rotating linear motion may be relative to, or along, a displacement axis. The linear relative motion may be a reciprocating motion.

The equipment may have a linear support that operatively connects, or operatively couples, the first element and the first magnets, or more specifically the first element and the second element. It is understood that the linear support slidably supports the first element relative to the first magnets, or vice versa, or more specifically that the linear support slidably supports the first element relative to the second element, or vice versa. The linear support may be arranged to allow the first element to reciprocate relative to the first magnets, or vice versa. More specifically linear support may be arranged to allow the first element to reciprocate relative to the second element, or vice versa

The first magnets, or the second element, may be static, or more precisely have a static position and/or orientation, and the first element may be movable, or more precisely have a dynamic position and/or orientation. It is understood that this is relative to the surroundings of the equipment and when operating the equipment.

Alternatively, the first element may be static, or more precisely have a static position and/or orientation, and the first magnets, or the second element, may be movable, or more precisely have a dynamic position and/or orientation. It is understood that this is relative to the surroundings of the equipment and when operating the equipment.

Alternatively, the first element may be movable, or more precisely have a dynamic position and/or orientation, and the first magnets, or the second element, may be movable, or more precisely have a dynamic position and/or orientation. It is understood that this is relative to the surroundings of the equipment and when operating the equipment.

It is specified that the relative motion may be a rotating relative motion relative to, or around, a rotational axis, or that the relative motion may be a linear relative motion relative to, or along, a displacement axis. It is further specified that the equipment comprises one or more first magnets. More specifically, the equipment may comprise a plurality of first magnets. The first magnets may be distributed along the rotational axis or the displacement axis. Additionally or alternatively, the first magnets may be distributed around the rotational axis or the displacement axis. More specifically, the first magnets may be evenly distributed around the rotational axis or the displacement axis. For example, there may be two first magnets that are positioned with a 180° separation between, there may be three first magnets that are positioned with a 120° separation between them, and there may be four first magnets that are positioned with a 90° separation between them. It is understood that this is relative to the rotational axis or the displacement axis.

It is specified that the equipment may comprise a second element. The equipment may comprise: one or more second magnets, the second element may be configured to be exposed to the fluid, and each of the second magnets is, or the second magnets are, arranged to form eddy currents in the second element at the relative motion. More specifically, each of the second magnets is, or the second magnets are, arranged to form eddy currents in the second element that inhibits, or reduces, microbiological growth on the second element at the relative motion.

The proposed method may further comprise: arranging, or providing, one or more second magnets to form eddy currents in the second element at the relative motion. More specifically, the method comprises: arranging, or providing, one or more second magnets to form eddy currents in the second element that inhibits, or reduces, microbiological growth on the second element at the relative motion.

It is understood that that each of the second magnets is arranged to form, or generate, a magnetic field. It is further understood that each of the second magnets is arranged to subject the second element, or at least a portion of the second element, to the magnetic field. It is further understood that each of the second magnets may be arranged to subject the complete second element to the magnetic field. It is further understood that the second magnets may be spaced apart from the second element. It is further understood that there may be a single second magnet.

The one or more second magnets may be stationary, or have a fixed position, relative to first element. The second magnets may be located at the second element. More specifically, the second magnets may be supported by, or attached to, the first element. Alternatively, the second magnets may be spaced apart from the first element. For example, the equipment may comprise a second magnet support that supports the second magnets relative to the first element. The second magnet support may space the second magnets apart from the first element.

The second magnets may be partly or fully embedded in the first element. The second magnets may be isolated from the fluid. Worded differently, the second magnets may be prevented from contacting the fluid. For example, the equipment may comprise a second cover that covers the second magnets and isolates the second magnets from the fluid. The second cover may be formed by the first element. Worded differently, the first element may be configured to prevent the fluid from contacting the second element. More specifically, the second magnets may be fully embedded in the first element. It is understood that the first element then covers the second magnets and prevents the fluid from contacting the second magnets.

It is specified that the relative motion may be a rotating relative motion relative to, or around, a rotational axis, or that the relative motion may be a linear relative motion relative to, or along, a displacement axis. It is further specified that the equipment comprises one or more second magnets. More specifically, the equipment may comprise a plurality of second magnets. The second magnets may be distributed along the rotational axis or the displacement axis. Additionally or alternatively, the second magnets may be distributed around the rotational axis or the displacement axis. More specifically, the second magnets may be evenly distributed around the rotational axis or the displacement axis. For example, there may be two second magnets that are positioned with a 180° separation between, there may be three second magnets that are positioned with a 120° separation between them, and there may be four second magnets that are positioned with a 90° separation between them. It is understood that this is relative to the rotational axis or the displacement axis.

It is specified that the relative motion may be a rotating relative motion. It is understood that the actuator, or the actuators, may be arranged to operate the equipment at relative motion having a rotational rate. It is further understood that the actuator, or the actuators, may be arranged to operate the equipment at relative motion having a relative speed between the first element and the first magnets at the first magnets. The rotational rate may be below 15 000 rpm or below 10 000 rpm. The rotational rate may be above 5 000 rpm. The relative speed may be below 320 m/s or below 160 m/s. The relative speed may be above 80 m/s. For example, this may be suitable for centrifuge separators in dairy applications. The rotational rate may be below 5 000 rpm or below 2 500 rpm. The rotational rate may be above 500 rpm. The relative speed may be below 80 m/s or below 40 m/s. The relative speed may be above 20 m/s. For example, this may be suitable for centrifugal pumps in dairy applications.

It is specified that the relative motion may be a linear relative motion. It is understood that the actuator, or the actuators, may be arranged to operate the equipment at relative motion having a relative mean speed between the first element and the first magnets at the first magnets. The relative mean speed may be below 2 m/s or below 1 m/s. For example, this may be for smaller high-speed pumps. The relative mean speed may be above 0.05 m/s or above 0.5 m/s. For example, this may be for larger low-speed pumps.

It is specified that the linear relative motion may be a reciprocating motion. It is understood that the actuator, or the actuators, may be arranged to operate the equipment at relative motion having a cycle rate. It is understood that the cycle rate is the number of complete strokes in one direction, for example one intake stroke and one discharge stroke, the first element or the second element makes per unit of time. The cycle rate may be below 120 complete strokes per minute or below 60 complete strokes per minute. The stroke rate may be above 30 complete strokes per minute.

It is specified that the actuator is, or the actuators are, arranged to cause a relative motion between the first element and the first magnets. The equipment may have an intended relative motion, or nominal relative motion, when operating the equipment. It is understood that the actuator, or the actuators, may be arranged to cause the intended relative motion at an average output power from the actuator, or the actuators. It is further understood that the actuator, or actuators, may be arranged to cause the intended relative motion at an average input power to the actuator, or the actuators. Worded differently, the actuator consumes an average input power when causing the intended relative motion. It is further specified that each of the first magnets are arranged to form eddy currents in the first element at the relative motion.

More than 2%, 4%, or 8% of the average output power may be converted to eddy currents by the first magnets. Worded differently, the average output power may be reduced by more than 2%, 4%, or 8% for maintaining the intended relative motion if the first magnets are removed. Less than 60%, 40%, or 20% of the average output power may be converted to eddy currents by the first magnets. Worded differently, the average output power may be reduced by less than 60%, 40%, or 20% for maintaining the intended relative motion if the first magnets are removed.

More specifically, the actuator may be an electric motor, or the actuators may be electric motors. The electric motor or electric motors may cause the relative motion at an average input power to the electric motor or electric motors. It is understood that this is when operating of the equipment. It is assumed that the electric motor operates, or the electric motors jointly operate, at a certain efficiency, and that there may be mechanical power losses in the coupling between the actuator, or the actuators, and the rest of the equipment. More than 1%, 2%, or 4% of the average input power may be converted to eddy currents by the first magnets. Worded differently, the average input power may be reduced by more than 1%, 2%, or 4% for maintaining the intended relative motion if the first magnets are removed. Less than 30%, 20%, or 10% of the average input power may be converted to eddy currents by the first magnets. Worded differently, the average input power may be reduced by less than 30%, 20%, or 10% for maintaining the intended relative motion if the first magnets are removed.

It is specified above that the equipment may comprise one or more second magnets, and that each of the second magnets are arranged to form eddy currents in the first element at the relative motion. More than 2%, 4%, or 8% of the average output power may be converted to eddy currents by the first magnets and the second magnets. Worded differently, the average output power may be reduced by more than 2%, 4%, or 8% for maintaining the intended relative motion if the first magnets and the second magnets are removed. Less than 60%, 40%, or 20% of the average output power may be converted to eddy currents by the first magnets and the second magnets. Worded differently, the average output power may be reduced by less than 60%, 40%, or 20% for maintaining the intended relative motion if the first magnets and the second magnets are removed.

More specifically, the actuator may be an electric motor, or the actuators may be electric motors. The electric motor or electric motors may cause the relative motion at an average input power to the electric motor or electric motors. It is understood that this is when operating of the equipment. It is assumed that the electric motor operates, or the electric motors jointly operate, at a certain efficiency, and that there may be mechanical power losses in the coupling between the actuator, or the actuators, and the rest of the equipment. More than 1%, 2%, or 4% of the average input power may be converted to eddy currents by the first magnets and the second magnets. Worded differently, the average input power may be reduced by more than 1%, 2%, or 4% for maintaining the intended relative motion if the first magnets and the second magnets are removed. Less than 30%, 20%, or 10% of the average input power may be converted to eddy currents by the first magnets and the second magnets. Worded differently, the average input power may be reduced by less than 30%, 20%, or 10% for maintaining the intended relative motion if the first magnets and the second magnets are removed.

Each of the first magnets may be arranged to form, or generate, a magnetic field at the first element that has a flux density greater than 0.5 mT, 1 mT, 10 mT, or 100 mT. Similarly, each of the second magnets may be arranged to form, or generate, a magnetic field at the second element that has a flux density greater than 0.5 mT, 1 mT, 10 mT, or 100 mT.

The first element may be electrically conductive. The first element may be non-magnetic. The first element may be of a first material. The first material may be a metal. The first material may have a magnetic relative permeability that is less than 2000, or less than 1000. For example, the first material may be stainless steel, such as ferritic stainless steel or martensitic stainless steel. Preferably, the first material is non-magnetic. For example, it may be austenitic stainless steel or an aluminium alloy. It may have a magnetic relative permeability that is less than 50, less than 10, or less than 5. This will contribute to reduce the influence of the first magnets on the operation of the equipment. Alternatively, the first material may have a magnetic relative permeability that is greater than 2000, or greater than 4000. For example, the second material may be cast steel or cast iron.

The second element may be electrically conductive. The second element may be non-magnetic. The second element may be of a second material. The second material may be a metal. The second material may have a magnetic relative permeability that is less than 2000, or less than 1000. For example, the second material may be stainless steel, such as ferritic stainless steel or martensitic stainless steel. Preferably, the second material is non-magnetic. For example, it may be austenitic stainless steel or an aluminium alloy. It may have a magnetic relative permeability that is less than 50, less than 10, or less than 5. This will contribute to reduce the influence of the second magnets on the operation of the equipment. Alternatively, the second material may have a magnetic relative permeability that is greater than 2000, or greater than 4000. For example, the second material may be cast steel or cast iron.

It is understood that each of the first magnets is arranged to form, or generate, a magnetic field. The first element may have a first wall. It is understood that the first wall may be configured to be exposed to the fluid. The first magnets, or one or more of the first magnets, may be arranged to form eddy currents in the first wall at the relative motion. The wall may be located between the first magnets and the fluid. The first element and the first magnets may be arranged to allow the magnetic fields to partly pass through, or at least partly pass through, the first wall at the relative motion between the first element and the first magnets.

The first magnets may be positioned pairwise so that the magnetic fields of the first magnets of each pair are aligned at the first element, or the first wall. For example, the first magnets of each pair may be oriented with opposite poles facing, or opposite polarities relative to, the first element. It is specified that the first magnets may be located at the second element. The second element may be arranged to lead the magnetic field between the magnets of each pair. As specified above, the second material of the second element may have a magnetic relative permeability that is greater than 2000, or greater than 4000, and it may be of cast steel or cast iron.

Similarly, the second magnets may be positioned pairwise so that the magnetic fields of the second magnets of each pair are aligned at the second part. For example, the second magnets of each pair may be oriented with opposite poles facing, or opposite polarities relative to, the first element.

The first magnets and/or the second magnets may be permanent magnets. The permanent magnets may be ferrite magnets, alnico magnets, or rare-earth magnets, such as neodymium magnets. More specifically, the rare-earth magnets may be samarium-cobalt magnets or neodymium-iron-boron magnets.

The first magnets and/or the second magnets may be electromagnets. Each of the electromagnets may comprise a coil arranged to generate the magnetic field at a current through the coil. The equipment may comprise a power source, or power source arrangement, arranged to supply each of the coils with a current. It is understood that this is for forming the magnetic fields. The electromagnets have the advantage over the permanent magnets in that they can be operated intermittently, which may contribute to reducing unnecessary power losses when operating the equipment.

The electromagnets, or coils, may extend around, or encircle, the first element. Additionally or alternatively, the electromagnets, or coils, may extend around, or encircle, the second element. Worded differently, the first element may be located within the electromagnets, or coils. Additionally or alternatively, the second element may be located within the electromagnets, or coils. It is understood that each electromagnet, or coil, may have a magnetic axis that extends through the center of the electromagnet, or coil. The magnetic axis of each coil may be parallel with the rotational axis or displacement axis. More specifically, each electromagnet, or coil, may be positioned and/or oriented with its magnetic axis parallel with the rotational axis or displacement axis. The electromagnets, or coils, may be centered on the rotational axis or displacement axis. The magnetic axis of each coil may be colinear with, or coincide with, the rotational axis or displacement axis. More specifically, each electromagnet, or coil, may be positioned and/or oriented with its magnetic axis being colinear with, or coinciding with, the rotational axis or displacement axis.

It is specified that the first magnets, or the second element, may be static, and that the first element may be movable. It is further specified that the relative motion may be a rotating relative motion. The first element may be, or comprise, a rotating element. The second element may be, or comprise, a housing, or a container.

Alternatively, it is specified that the first magnets, or the second element, may be movable, and that the first element may be static. It is further specified that the relative motion may be a rotating relative motion. The first element may be, or comprise, a housing, or a container. The second element may be, or comprise, a rotating element.

The housing may be configured to hold, or contain, the fluid. More specifically, the housing may form, or enclose, an inner space for holding, or retaining, the fluid, for example in centrifugal pumps and extruders. The rotating element may be located within, or at least partly within, the housing. The inner side of the housing may face the inner space and/or the rotating element. The housing may have an inside and an outside. More specifically, the housing may have an inner side and an outer side. The rotating element may be located on, or at, the inside, or the inner side, of the housing. It is understood that the housing may at least partly enclose rotating element.

Additionally or alternatively, the rotating element may be configured to hold, or contain, the fluid, for example in centrifuge separators. More specifically, the rotating element may form, or enclose, an inner space for holding, or retaining, the fluid. The rotating element may be located within, or at least partly within, the housing. If the first element or the second element is static, it may comprise an inner part that is located within, or extends into, the rotating element, or more specifically the inner space of the rotating element, for example in centrifuge separators. It is understood that the inner part may be connected to the housing.

As specified above, the first magnets may be located at the second element, the second magnets may be located at the first element, the first magnets may be supported by the second element, and the second magnets may be supported by, or attached to, the first element. If the first element is a rotating element and the second element is a housing and/or an inner part, the first magnets may be located at the housing and/or the inner part, and the first magnets may be supported by, or attached to, the housing and/or an inner part. Additionally, the second magnets may be located at the rotating element, and the second magnets may be supported by, or attached to, the rotating element. If the first element is a housing and/or an inner part and the second element is a rotating element, the first magnets may be located at the rotating element, and the first magnets may be supported by, or attached to, the rotating element. Additionally, the second magnets may be located at the housing and/or an inner part, and the second magnets may be supported by, or attached to, the housing and/or the inner part. In the applications described below, it is understood that the first magnets and the second magnets may be positioned on any of the described parts of the second element respective the first element.

The equipment may comprise, or be, a centrifugal pump and the first element and the second element, or more specifically the rotating element and the housing, form part of the centrifugal pump. The housing may have, or form, an inlet and an outlet. It is understood that the inlet is for receiving, or introducing, the fluid into the housing, and the outlet is for discharging, or dispelling, the fluid from the housing. The outlet may be located off-axis relative to the rotational axis. The inlet may be centred on the rotational axis.

The housing, or the inner space, may form a volute, or curved funnel with a cross-section that increases towards the outlet. Worded differently, the housing may comprise, or be, a volute casing that forms a volute. The rotating element may comprise an impeller and a shaft, wherein the impeller is attached to, or fixed to, the shaft and arranged to, or configured to, generate a flow of the fluid from the inlet to the outlet at the rotating relative motion. It is understood that the impeller and the shaft may be centred on the rotational axis. It is understood that the impeller is located within the volute or volute casing. It is understood that the shaft may extend from inside the housing to outside the housing, or that it is accessible from outside the housing. The actuator may be operationally connected to the shaft. The actuator may be located outside the housing. For example, the actuator may be an electric motor, and the electric motor may be connected directly to the shaft. It is understood that the first magnets or the second magnets may be supported by, or attached to, the impeller and/or the shaft.

The equipment may comprise, or be, an axial-flow pump and the first element and the second element, or more specifically the rotating element and the housing, form part of the axial-flow pump. The housing may have, or form, an inlet and an outlet. It is understood that the inlet is for receiving, or introducing, the fluid into the housing, and the outlet is for discharging, or dispelling, the fluid from the housing. The inlet and outlet may be centred on the rotational axis. Worded differently, the housing may form a straight tube between the inlet and the outlet. Alternatively, the inlet may be centred on the rotational axis and the outlet may be located off-axis relative to the rotational axis, or the outlet may be centred on the rotational axis and the inlet may be located off-axis relative to the rotational axis. Worded differently, the housing may form a bent tube between the inlet and the outlet.

The rotating element may comprise an impeller and a shaft, wherein the impeller is attached to, or fixed to, the shaft and arranged to, or configured to, generate a flow of the fluid from the inlet to the outlet at the rotating relative motion. It is understood that the impeller and the shaft may be centred on the rotational axis. It is understood that the impeller is located within the housing, or more specifically within the straight or bent tube formed by the housing. It is understood that the shaft may extend from inside the housing to outside the housing, or that it is accessible from outside the housing. The actuator may be operationally connected to the shaft. The actuator may be located outside the housing. For example, the actuator may be an electric motor, and the electric motor may be connected directly to the shaft. It is understood that the first magnets or the second magnets may be supported by, or attached to, the impeller and/or the shaft.

The equipment may comprise, or be, an extruder, and the first element and the second element, or more specifically the rotating element and the housing, form part of the extruder. The housing may have, or form, an inlet and an outlet. It is understood that the inlet is for receiving, or introducing, the fluid into the housing, and the outlet is for discharging, or dispelling, the fluid from the housing. The inlet may be, or have, a feed nozzle or a hopper. It may be located off-axis relative to the rotational axis. The outlet may be a discharge nozzle or die. It may be centred on the rotational axis.

The housing may comprise, or be, a casing or a barrel. The rotating element may comprise a rotor, or screw, and a shaft, wherein the rotor is attached to, or fixed to, the shaft and arranged to, or configured to, generate a flow of the fluid from the inlet to the outlet or to push the fluid, at the rotating relative motion. It is understood that the rotor and the shaft may be centred on the rotational axis. It is understood that the rotor is located within casing. It is understood that the shaft may extend from inside the housing to outside the housing, or that it is accessible from outside the housing. The actuator may be operationally connected to the shaft. The actuator may be located outside the housing. For example, the actuator may be an electric motor, and the electric motor may be coupled to the shaft. It is understood that the first magnets or the second magnets may be supported by, or attached to, the roto and/or the shaft.

The equipment may comprise, or be, a rotary-type positive-displacement pump, and the first element and the second element, or more specifically the rotating element and the housing, form part of the rotary-type positive-displacement pump. The housing may have, or form, an inlet and an outlet. It is understood that the inlet is for receiving, or introducing, the fluid into the housing, and the outlet is for discharging, or dispelling, the fluid from the housing. For example, the inlet and the outlet may be off-axis relative to the rotational axis. The positions of the inlet and outlet depend on the type of pump.

For example, the rotary-type positive-displacement pump may be a single-screw pump. The rotating element may then comprise a screw and a shaft, and the screw is attached to, or fixed to, the shaft and arranged to, or configured to, generate a flow of the fluid from the inlet to the outlet at the rotating relative motion. It is understood that the screw and the shaft may be centred on the rotational axis. It is understood that the screw is located within the housing. It is understood that the shaft may extend from inside the housing to outside the housing, or that it is accessible from outside the housing. The actuator may be operationally connected to the shaft. The actuator may be located outside the housing. For example, the actuator may be an electric motor, and the electric motor may be coupled to, or connected directly to, the shaft. The housing may comprise, or form, a stator, and the screw and the stator may be arranged to, or configured to, cooperate and generate the flow of the fluid from the inlet to the outlet. It is understood that the first magnets or the second magnets may be supported by, or attached to, the screw, shaft and/or the stator.

Alternatively, the rotary-type positive-displacement pump may be a vane pump. The rotating element may then comprise a rotor and a shaft, and the rotor is attached to, or fixed to, the shaft and arranged to, or configured to, generate a flow of the fluid from the inlet to the outlet at the rotating relative motion. It is understood that the rotor and the shaft may be centred on the rotational axis. It is understood that the rotor is located within the housing. The actuator may be operationally connected to the shaft. For example, the actuator may be an electric motor, and the electric motor may be connected directly to the shaft. The rotor may have, or comprise, vanes. The vanes may have a variable length and/or be biased, or tensioned, to maintain contact with the housing. It is understood that the vanes may extend radially relative to the shaft or the rotational axis. It is further understood that the vanes are arranged to, or configured to, generate the flow of the fluid from the inlet to the outlet. It is understood that the first magnets or the second magnets may be supported by, or attached to, the rotor and/or the shaft.

Alternatively, the rotary-type positive-displacement pump may be a progressing cavity pump. The rotating element may then comprise a helical rotor and the housing may comprise, or form, a stator having a helical hole, or bore, in which the helical rotor is located. It is understood that the helical rotor and the stator are arranged to cooperate to pump the fluid from the inlet to the outlet at the rotating relative motion of the rotating element.

Alternatively, the rotary-type positive displacement pump may be a shuttle block pump. The rotating element may then comprise a rotor, a piston, and a shuttle. It is understood that these components are arranged to cooperate to pump the fluid from the inlet to the outlet at the rotating relative motion of the rotating element.

Alternatively, the rotary-type positive-displacement pump may be a dual-rotor pump that comprises a rotor and an additional rotor. The rotating element may then comprise the rotor. It is understood that the rotor and the additional rotor are arranged to cooperate to pump the fluid from the inlet to the outlet at the rotating relative motion of the rotating element. For example, the dual-rotor pump may be a lobe pump, and the rotor and the additional rotors are a lobe rotor respectively an additional lobe rotor; or the dual-rotor pump may be a circumferential piston pump, and the rotor and the additional rotors are a winged rotor respectively an additional winged rotor.

Alternatively, the rotary-type positive displacement pump may be a multi-screw pump, for example a twin-screw pump or a three-screw pump that comprises a plurality of screws. The rotating element may then comprise one of the screws. It is understood that the screws are arranged to cooperate to pump the fluid from the inlet to the outlet at the rotating relative motion of the rotating element.

The equipment may comprise, or be, a mixer, or agitator and the first element and the second element, or more specifically the rotating element and the housing, form part of the mixer. The housing may have an inlet and an outlet. The inlet and the outlet may be located off-axis relative to the rotational axis. A mixer is understood to be configured for agitating a fluid, for example to mix two fluid components, or to mix a fluid component with a solid particulate component.

The rotating element may comprise an agitator, or rotor and a shaft, wherein the agitator is attached to, or fixed to, the shaft and arranged to, or configured to, agitate, or stir, the fluid held, or contained, by the housing at a rotation of the rotating element at the rotating relative motion. It is understood that the agitator and the shaft may be centred on the rotational axis. It is understood that the agitator is located within the housing, or the inner space. The actuator may be operationally connected to the shaft. For example, the actuator may be an electric motor, and the electric motor may be coupled to the shaft.

The equipment may comprise, or be, a centrifuge separator, and the first element and the second element, or more specifically the rotating element and the housing, form part of the centrifuge separator. It is understood that centrifuge separators encompass centrifuge clarifiers. It is further understood that the properties, or composition, of the fluid, or liquid, introduced into the centrifuge separator may differ from those of the fluids, or liquids, discharged from the centrifuge separator at the rotating relative motion. The properties, or composition, may also vary within the rotating element. For example, the centrifuge separator may form lighter and heavier phases of the fluid, or liquid, at the rotating relative motion. It is understood that the centrifuge separator may be arranged to discharger the lighter and heavier phases separately from one another.

It is specified that the rotating element may be configured to hold the fluid, and that it may form an inner space for holding the fluid. The rotating element may comprise a bowl, or a rotor body, configured to hold, or contain, the fluid. More specifically, the bowl may form, or enclose, the inner space. It is understood that the rotating element, or bowl, may be an assembled structure, for example it may have a lower bowl body and an upper bowl body. Here, the positions of the lower bowl body and the upper bowl body are relative to a vertical rotational axis. The lower bowl body and the upper bowl body may be annular and centred on the rotational axis. Additionally, they may have rotational symmetry, or be rotationally symmetric, relative to the rotational axis. The lower bowl body and the upper bowl body may form, or enclose, the abovementioned inner space. Alternatively, the rotating element, or the bowl, may further have a sliding piston, or sliding bowl bottom, arranged to slide relative to the lower bowl body parallel to the rotational axis. It is understood that the sliding piston is located within the combined lower bowl body and upper bowl body. The sliding piston and the upper bowl body may form, or enclose, the abovementioned inner space. The sliding piston may have an upper position in which it seals to the upper bowl body and a lower position in which the fluid, or sludge accumulated from the fluid, can be ejected from the inner space between the upper bowl body and the sliding piston. The lower bowl body may form a plurality of ejection ports, or sludge ejection ports, through which fluid, or accumulated sludge, can pass after being ejected from the inner space.

The rotating element, or the bowl, may have a rotational symmetry, or be rotationally symmetric, relative to the rotational axis. It is specified that the rotating element may be located within, or at least partly within, the housing. More specifically, the bowl may be located within, or at least partly within, the housing. Worded differently, the housing may enclose the bowl.

The rotating element may comprise a shaft, or spindle, wherein the bowl, or the lower bowl body, is attached to, or fixed to, the shaft. The shaft may extend from within the housing to outside the housing. It is understood that the bowl and the shaft may be centred on the rotational axis. It is understood that the shaft may extend from inside the housing to outside the housing, or that it is accessible from outside the housing. The actuator may be operationally connected to the shaft. The actuator may be located outside the housing. For example, the actuator may be an electric motor, and the electric motor may be coupled to, or connected directly to, the shaft.

The rotating element may have, or form, an inlet. It is understood that the inlet is for receiving, or introducing, the fluid into the rotating element, or more precisely into the bowl or interior space. The inlet may be formed by the shaft, and the shaft may form a fluid conduit that connects the inlet to the bowl or the inner space.

Alternatively, the inlet may be located within the bowl or in the inner space. It is specified that the first element or the second element may comprise an inner part that is located within the rotating element or the inner space of the rotating element, and that the inner part may be connected to the housing. For example, the inner part may be, or comprise, an inlet pipe that extends from outside the rotating element, or bowl, into the rotating element, the bowl, or the inner space. The inlet pipe may be centred on the rotating axis. The inlet pipe may have an outer open end for receiving the fluid and an inner open end at the inlet. It is understood that the outer open end is located outside the rotating element and the inner open end is located inside the rotating element, the bowl, or the inner space.

The rotating element may have an outlet. The inlet and the outlet may be located at opposite sides, or ends, of the rotating element, or the bowl. Alternatively, the inlet and the outlet may be located at the same side, or end, of the rotating element, or the bowl. Additionally, the rotating element may have an additional outlet. The inlet and the additional outlet may be located at opposite sides, or ends, of the rotating element, or the bowl. Alternatively, the inlet and the additional outlet may be located at the same side, or end, of the rotating element, or the bowl.

The centrifuge separator may be arranged to form a lighter phase, or low-density fraction, of the fluid and a heavier phase, or a high-density fraction, of the fluid within the rotating element, or in the inner space, at the rotating relative motion. The centrifuge separator may be configured to discharge the lighter phase of the fluid through the outlet. It may further be configured to discharge the heavier phase of the fluid through the additional outlet. Additionally or alternatively, it may further be arranged to discharge the heavier phase of the fluid through the abovementioned ejection ports.

The rotating element and the housing may jointly form a pump arranged to pump the fluid through the outlet at the rotating relative motion. For example, the rotating element may comprise a paring chamber and the housing may comprise a paring disc that jointly forms a centripetal pump arranged to pump the fluid through the outlet at the rotating relative motion; or the rotating element may comprise an impeller and the housing may comprise a volute, or volute casing, that jointly forms a centrifugal pump arranged to pump the fluid through the outlet at the rotating relative motion.

Similarly, the rotating element and the housing may jointly form an additional pump arranged to pump the fluid from the through the additional outlet at the rotating relative motion. For example, the rotating element may comprise an additional paring chamber and the housing may comprise an additional paring disc that jointly forms an additional centripetal pump arranged to pump the fluid through the additional outlet at the rotating relative motion; or the rotating element may comprise an additional impeller and the housing may comprise an additional volute, or additional volute casing, that jointly forms an additional centrifugal pump arranged to pump the fluid through the additional outlet at the rotating relative motion.

The rotating element may comprise a partition, or top disc, arranged to guide, or lead, the lighter phase of the fluid to, or towards, the outlet or the pump, and the heavier phase to, or towards, the additional outlet or the additional pump. It is understood that the partition is located within, or at least partly within, the bowl, or in, or at least partly in, the inner space. The partition may be arranged to separate the lighter phase of the fluid from the heavier phase of the fluid.

The partition may be rotationally locked to, of fixed to, the bowl, or more specifically the upper bowl body. The partition may be annular and centred on the rotational axis. It may be located at the upper bowl body and arranged to allow the heavier phase to pass between the partition and the upper bowl body.

The rotating element may comprise a plurality of discs, or bowl discs, configured to enhance the formation of the lighter phase and the heavier phase of the fluid. It is understood that the discs are located within the bowl or in the inner space. The discs may be annular and centred on the rotational axis. The discs may be arranged in a disc stack that is aligned with the rotating axis. Each disc may outline a truncated cone, or be frusto-conical, with a narrow end facing upward and a wide end facing downward. Each disc may have a central aperture at the distributer further described below, or at its narrow end. The core of the distributer, which is further specified below, may extend through the aperture. Each pair of neighbouring discs may form an intermediate space between them. It is understood that the fluid is present in the intermediate space in operation, or more precisely that the fluid can flow in the intermediate space at the rotating relative motion.

The rotating element may comprise a distributer, or disc carrier, arranged to, or configured to, distribute a flow of the fluid received via the inlet within the bowl or in the inner space. It is understood that the distributer is located within, or at least partly is located within, the bowl, or in, or at least partly in, the inner space. The distributer may have a rotational symmetry relative to the rotational axis. The distributer may be arranged to, or configured to, lead a flow of the fluid to the plurality of discs, or to the disc stack. The distributer may have, of form, a distributer cone arranged to, or configured to, face a flow of the fluid from the inlet. The distributer may be rotationally locked, of fixed to, the bowl or to the lower bowl body.

The distributer may support the abovementioned discs or disc stack. More specifically, the distributor may have, or form, a core extending through each disc and aligning the disc stack with the rotational axis. It is understood that the core is centred on the rotational axis and that the core may contact the discs. The core may form a plurality of radial fins, or radial ribs, that extend along the rotational axis, wherein each radial fin contacts the discs. Each pair of radial fins may form a gap between them that allows passage of the fluid from the disc stack, or from the intermediate space between each pair of neighbouring discs, toward to the outlet.

The inner space may be divided into chambers. It is further understood that the chambers may be in fluid communication with one another. The distributer may form, or delimit, an inlet chamber at the rotational axis that is in fluid communication with the inlet. The distributer and the bowl may jointly form, or delimit, a separation chamber that is in fluid communication with the inlet chamber. If the partition is present, the distributer, and partition may jointly form, or delimit, a separation chamber. The separation chamber may surround the inlet chamber, or it may be located radially outside the inlet chamber. It is understood that the abovementioned discs are located in the separation chamber. The distributer may form radially extending channels by which the inlet chamber is in fluid communication with the separation chamber. If the rotating element has no partition, the bowl and the distributer may jointly form, or delimit, an outlet chamber that is in fluid communication with the separation chamber and the outlet. If the rotating element has a partition, the partition and the distributer may jointly form, or delimit, an outlet chamber that is in fluid communication with the separation chamber and the outlet. The outlet chamber may be located above the inlet chamber, at the rotating axis, and/or radially inside the separation chamber. The partition and the bowl may jointly form, or delimit, an additional outlet chamber that is in fluid communication with the separation chamber and the additional outlet. The additional outlet chamber may be located above the inlet chamber, above the outlet chamber, and/or radially outside the outlet chamber.

It is understood that that the abovementioned first element may comprise the bowl. It is specified that the first element may have a first wall. The first wall may form part of the bowl. More specifically, it is understood that the first wall is configured to be exposed to the fluid. The first magnets, or one or more of the first magnets, may be arranged to form eddy currents in the bowl at the rotational relative motion. The bowl may be located between the first magnets and the fluid. The bowl and the first magnets may be arranged to allow the magnetic fields to partly pass through, or at least partly pass through, the first wall at the rotational relative motion. The bowl and the first magnets may be arranged to allow the magnetic fields to form eddy currents in a component, or one or more components, inside the bowl, or in the inner space, for example in the impeller, partition, disc stack, distributer, and/or core.

It is specified above that the first magnets may be located at and/or supported by the housing and/or the inner part. For example, this may be at or by the inlet pipe. At least one of the first magnets may be arranged to form eddy currents in at least a portion of, or the complete, bowl at the rotational relative motion. At least one of the first magnets may be arranged to form eddy currents in at least a portion of, or the complete, paring chamber at the rotational relative motion. At least one of the first magnets may be arranged to form eddy currents in at least a portion of, or the complete, impeller at the rotational relative motion. At least one of the first magnets may be arranged to form eddy currents in at least a portion of, or the complete, partition at the rotational relative motion. At least one of the first magnets may be arranged to form eddy currents in at least a portion of, or the complete, disc stack at the rotational relative motion. At least one of the first magnets may be arranged to form eddy currents in at least a portion of, or the complete, distributer at the rotational relative motion. At least one of the first magnets may be arranged to form eddy currents in at least a portion of, or the complete, core at the rotational relative motion. It is understood that the second magnets may be arranged as described here.

It is specified above that the second magnets may be located at and/or supported by the rotating element. At least one of the second magnets may be arranged to subject at least a portion of, or the complete, inner part, or the inlet pipe, to a magnetic field at the rotational relative motion. At least one of the second magnets may be arranged to subject at least a portion of, or the complete, paring disc to a magnetic field at the rotational relative motion. At least one of the first magnets may be arranged to subject at least a portion of, or the complete, volute to a magnetic field at the rotational relative motion. It is understood that the first magnets may be arranged as described here.

It is specified that the first magnets, or the second element, may be movable, and the first element may be movable. It is further specified that the relative motion may be a rotating relative motion.

It is understood that the first element and the second element may be arranged to rotate in the same direction or in opposite directions. If the first element and the second rotate in the same direction, it is understood that they may be arranged to rotate at different rates. If the first element and the second element rotate in the opposite directions, it is understood that they may be arranged to rotate at the same rate or different rates.

The first element may be, or comprise, a rotating element, and the second element may be, or comprise, a rotating housing, or a rotating container. Alternatively, the first element may be, or comprise, a rotating housing, or a rotating container, and the second element may be, or comprise, a rotating element.

The rotating housing may be configured to hold, or contain, the fluid. More specifically, the rotating housing may form, or enclose, an inner space for holding, or retaining, the fluid, for example in decanter centrifuges. The rotating element may be located within, or at least partly within, the rotating housing. The inner side of the rotating housing may face the inner space and/or the rotating element. The rotating housing may have an inside and an outside. More specifically, the rotating housing may have an inner side and an outer side. The rotating element may be located on, or at, the inside, or the inner side, of the rotating housing. It is understood that the rotating housing may at least partly enclose rotating element.

As specified above, the first magnets may be located at the second element, the second magnets may be located at the first element, the first magnets may be supported by, or fixed to, the second element, and the second magnets may be supported by, or fixed to, the first element. The first magnets may be located at the rotating housing, and the first magnets may be supported by, or fixed to, the rotating housing. Additionally, the second magnets may be located at the rotating element, and the first magnets may be supported by, or fixed to, the rotating element. Alternatively, the first magnets may be located at the rotating element, and the first magnets may be supported by, or fixed to, the rotating element. Additionally, the second magnets may be located at the rotating housing, and the second magnets may be supported by, or fixed to, the rotating housing. In the applications described below, it is understood that the first magnets and the second magnets may be positioned on any of the described parts of the second element respective the first element.

The equipment may comprise, or be, a decanter, or decanter centrifuge, and the first element and the second element, or more specifically the rotating element and the rotating housing, form part of the decanter. It is understood that that the properties, or composition, of the fluid introduced into the decanter may differ from those of the fluids discharged from the decanter at the rotating relative motion. The properties, or composition, may also vary within the rotating housing. For example, the decanter may form a lighter phase, or low-density fraction, of the fluid and a heavier phase, or a high-density fraction, of the fluid within the rotating housing, or in the inner space, at the rotating relative motion. It is understood that the decanter may be arranged to discharger the lighter phase and the heavier phase separately from one another.

It is specified that the rotating housing may be configured to hold the fluid, and that it may form an inner space for holding the fluid. The rotating housing may comprise a casing, or barrel, configured to hold, or contain, the fluid. More specifically, the casing may form, or enclose, the inner space.

The decanter may have, or form, an inlet. For example, the inlet may be formed by the rotating element. It is understood that the inlet is for receiving, or introducing, the fluid into the rotating housing, or more precisely the casing or interior space. It is further understood that the inlet may be located within the rotating housing or in the inner space. The inlet may be centred on, or located at, the rotational axis.

For example, the decanter may comprise, an inlet pipe that extends from outside the rotating housing into the rotating housing, or the inner space. It is understood that the inlet pipe is arranged to allow the fluid to be received, or introduced into the rotating housing, or more precisely the casing or interior space, via the inlet pipe. If the rotating element forms the inlet, the inlet pipe may extend from outside the rotating housing into the rotating element.

The inlet pipe may be centred on the rotating axis. The inlet pipe may have an outer open end for receiving the fluid and an inner open end at the inlet, or within the rotating element. It is understood that the outer open end is located outside the rotating housing and the inner open end is located inside the rotating housing, or the inner space. If the rotating element forms the inlet, it is understood that the inlet pipe, or the inner open end, may be in fluid communication with the inlet.

The rotating housing may have, or form, an outlet. Additionally, the rotating element may have, or form, an additional outlet. The inlet and the additional outlet may be located at opposite sides, or ends, of the rotating housing, or the casing. The inlet may be located between the outlet and the additional outlet.

It is understood that the outlet and the additional outlets are for discharging, or dispelling, the fluid from the rotating housing. The outlet and/or the additional outlet may be located off-axis relative to the rotational axis.

It is specified that the rotating housing may be configured to hold the fluid, and that it may form an inner space for holding the fluid. The rotating housing may have a first end and an opposite second end, wherein the first end and the second end along the rotational axis. The outlet may be located at the first end and the additional outlet may be located at the second end. The rotating housing, or the inner space, may outline a cylinder centred on the rotational axis at the first end, and it may taper towards the rotational axis at the second end.

The rotating element may comprise a rotor, scroll, or conveyer. The rotating element may comprise a rotating-element shaft, and the rotor may be attached to, or fixed to, the rotating-element shaft. The rotating housing may comprise a rotating-housing shaft, and the casing may be attached to, or fixed to, the rotating-hosing shaft. It is understood that each of the shafts may be a driven shaft, and that they may be centred on the rotational axis.

It is understood that the rotating-element shaft may extend from inside the rotating housing to outside the rotating housing, or that it is accessible from outside the rotating housing. It is specified that the equipment may comprise a plurality of actuators. The plurality of actuators may include a first actuator and a second actuator. The first actuator may be coupled to the rotating-element shaft. The first actuator may be located outside the rotating housing. For example, the first actuator may be an electric motor, and the electric motor may be connected directly to the rotating-element shaft, or it may be coupled via a gear arrangement. The second actuator may be coupled to the rotating-housing shaft. For example, the second actuator may be an electric motor, and the electric motor may be connected directly to the rotating-housing shaft, or it may be coupled via a gear arrangement.

The rotating-element shaft may be located at the first end and the rotating-housing shaft may be located at the second end. The inlet pipe may be located at the second end.

It is understood that the rotating housing is arranged to form a lighter phase, or low-density fraction, of the fluid and a heavier phase, or a high-density fraction, of the fluid within the rotating housing, or in the inner space, at the rotating relative motion. It is further understood that the rotor is arranged to, or configured to, generate a flow of the heavier phase of the fluid, or to push the heavier phase of the fluid, to the additional outlet, towards the second end of the rotating housing, or along the rotational axis, at the rotating relative motion.

It is specified that the first magnets, or the second element, may be static, and the first element may be movable. It is further specified that the relative motion may be a linear relative motion. The first element may be, or comprise, a reciprocating element. The second element may be, or comprise, a housing, or a container.

Alternatively, it is specified that the first magnets, or the second element, may be movable, and the first element may be static. It is further specified that the relative motion may be a linear relative motion. The first element may be, or comprise, a housing, or a container. The second element may be, or comprise, a reciprocating element.

The housing may be configured to hold, or contain, the fluid. More specifically, the housing may form, or enclose, an inner space for holding, or retaining, the fluid, for example in reciprocating pump applications. The reciprocating element may be located within, or at least partly within, the housing, or the inner space.

As specified above, the first magnets may be located at the second element, the second magnets may be located at the first element, the first magnets may be supported by, or fixed to, the second element, and the second magnets may be supported by, or fixed to, the first element. If the first element is a reciprocating element and the second element is a housing, the first magnets may be located at the housing, and the first magnets may be supported by, or fixed to, the housing. Additionally, the second magnets may be located at the reciprocating element, and the second magnets may be supported by, or fixed to, the reciprocating element. If the first element is a housing, the first magnets may be located at the reciprocating element, and the first magnets may be supported by, or fixed to, the reciprocating element. Additionally, the second magnets may be located at the housing, and the second magnets may be supported by, or fixed to, the housing. In the applications described below, it is understood that the first magnets and the second magnets may be positioned on any of the described parts of the second element respective the first element.

In the applications described below, it is understood that the first magnets and the second magnets may be positioned on any of the described parts of the second element respective the first element.

The equipment may comprise, or be, a positive-displacement pump and the first element and the second element, or more specifically the reciprocating element and the housing, form part of the positive-displacement pump.

The housing may have, or form, an inlet, or feed nozzle, and an outlet, or discharge nozzle. It is understood that the inlet is for receiving, or introducing, the fluid into the housing, or the inner space, and the outlet is for discharging, or dispelling, the fluid from the housing, or the inner space. The inlet and the outlet may be located at the same side, or end, of the housing. It is understood that the reciprocating element is arranged to, or configured to, generate a flow of the fluid at the linear relative motion, or more specifically to generate a flow of the fluid into the housing, or the inner space, via the inlet and from the housing, or the inner space, via the outlet at the linear relative motion.

The positive-displacement pump may be a piston pump or a plunger pump. The reciprocating element may then be, or comprise, a piston respective a plunger.

In the piston pump, it is understood that the housing may comprise a cylinder block that forms a cylinder in which the piston can reciprocate. It is specified above that the equipment may have a linear support. It is understood that the cylinder block may form, or be arranged to form, the linear support. This means that the inner space is partly formed by the cylinder block. The housing may further comprise a cylinder head that is attached and sealed to the cylinder block, and the cylinder block and the cylinder head jointly form, or enclose, the inner space. The cylinder head may form the inlet and the outlet.

The equipment may comprise a shaft arrangement. The shaft arrangement may comprise a connecting rod and a crankshaft, wherein the crank shaft is connected to the piston or plunger via the connecting rod. It is understood that the crankshaft may be accessible from outside the housing. It is further understood that the crankshaft is operationally connected to the piston or plunger via the connecting rod and arranged to reciprocate the piston or plunger at a rotation of the crankshaft. It is further understood that the connecting rod is rotationally connected to the crankshaft and pivotally connected to the piston or plunger. It is further understood that the crankshaft and the connecting rod are arranged to convert a rotation of the crankshaft to a reciprocating linear relative motion between the piston or plunger and the housing. More specifically, the crankshaft may have, or form, a crank pin, the shaft arrangement may have a rod bearing, and the connecting rod may be connected to the crank pin via the rod bearing. For example, the rod bearing may be a rolling element bearing. The shaft arrangement may comprise a piston pin, or plunger pin, that pivotally connects the connecting rod to the piston or plunger. Worded differently, the connecting rod may be pivotally connected to the piston via the piston pin, or the connecting rod may be pivotally connected to the plunger via the plunger pin. It is understood that the crankshaft has a crankshaft axis and that it is arranged to rotate around the crankshaft axis at the linear relative motion. It is further understood that the crankshaft axis may be perpendicular to the displacement axis. The actuator may be an electric motor, and the crank may be configured to be operated, or rotated, by the electric motor.

Alternatively to the crankshaft, the shaft arrangement may comprise a linear rod, or push rod, arranged to reciprocate the piston or plunger at a reciprocating linear motion of the linear rod. It is understood that the linear rod has a linear-rod axis and that it is arranged to reciprocate along the linear-rod axis, or the displacement axis. The linear rod and the reciprocating element may be coaxial. Worded differently, the linear-rod axis and the displacement axis may be co-linear and overlapping. The actuator may be a linear actuator, such as an electromechanical actuator or a hydraulic cylinder, and the linear rod may be arranged to be operated by the linear actuator.

Alternatively, the shaft arrangement may further comprise a swashplate and a rotary shaft, wherein the swashplate operationally connects the linear rod to the rotary shaft and is arranged to reciprocate the linear rod at a rotation of the rotary shaft. It is understood that the rotary shaft has a rotary-shaft axis and that it is arranged to rotate around the rotary-shaft axis at the linear relative motion. The actuator may be an electric motor, and the rotary shaft may be configured to be operated by the electric motor. The rotary shaft may be off-axis relative to the reciprocating element. Worded differently, the rotary-shaft axis and the displacement axis may be parallel and nonoverlapping.

It is understood that the linear rod or rotary-shaft may extend from inside the housing to outside the housing, or that they are accessible from outside the housing. The actuator may be operationally connected to the linear rod or the rotary-shaft. The actuator may be located outside the housing. For example, the actuator may be connected directly to the linear rod or the rotary-shaft.

### Brief description of the drawings

A more complete understanding of the abovementioned and other features and advantages of the technology proposed herein will be apparent from the following detailed description of preferred embodiments in conjunction with the appended drawings, wherein:
Fig. 1 is a schematic cross section of a fluid-processing equipment in the form of a centrifugal pump,
Fig. 2 is a schematic cross section of another fluid-processing equipment in the form of a centrifugal pump,
Fig. 3 is a schematic cross section of another fluid-processing equipment in the form of a centrifugal pump,
Fig. 4 is a schematic cross section of a fluid-processing equipment in the form of an extruder,
Fig. 5 is a schematic cross section of another fluid-processing equipment in the form of an extruder,
Fig. 6 is a schematic cross section of fluid-processing equipment in the form of a centrifuge separator,
Fig. 7 is a schematic cross section of another fluid-processing equipment in the form of a centrifuge separator,
Fig. 8 is a schematic cross section of another fluid-processing equipment in the form of a centrifuge separator,
Fig. 9 is a schematic cross section of a fluid-processing equipment in the form of a decanter centrifuge, and
Fig. 10 is a schematic cross section of a fluid-processing equipment in the form of a piston pump.

### Detailed description

In the figures and the below description, the same reference numerals are used for the same or related features.

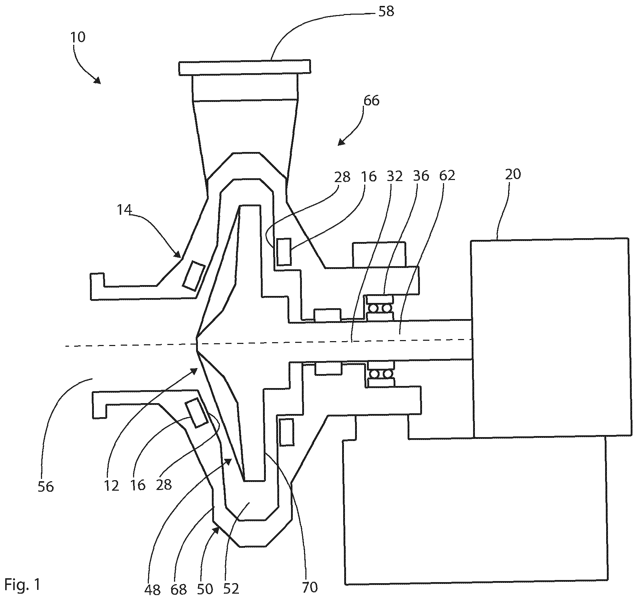

Fig. 1 shows an embodiment of a fluid-processing equipment 10 in the form of a centrifugal pump 66 for pumping a fluid, or more precisely a liquid. The centrifugal pump 66 has a housing 50 and a rotating element 48. The housing 50 is static and the rotating element 48 is movable relative to the surroundings. The rotating element 48 rotates around a rotational axis 32 when operating the centrifugal pump 66. The housing 50 has an inner space 52 for holding the fluid. This means that the housing 50 is configured to be exposed to the fluid. It further has an inlet 56 for receiving the fluid into the housing 50 and an outlet 58 for discharging the fluid from the housing 50. The inlet 56 is centred on the rotational axis 32, and the outlet 58 is located off-axis relative to the rotational axis 32.

The housing 50 has a volute casing 68 that forms a volute with cross-section that increases towards the outlet 58. The volute forms part of the inner space 52 of the housing 50. The rotating element 48 has an impeller 70 and a shaft 62 that are centred on the rotational axis 32, and the impeller 70 is attached to the shaft 62. The rotating element 48 is of a first material, and the first material is a non-magnetic austenitic stainless steel. The housing 50 is of a second material, and the second material is also a non-magnetic austenitic stainless steel.

The impeller 70 is located within the volute and is configured to generate a flow of the fluid from the inlet 56 to the outlet 58 at a rotating relative motion between the rotating element 48 and the housing 50. This means that the impeller 70 is configured to be exposed to the fluid within the housing 50.

The shaft 62 extends from inside the housing 50 to outside the housing 50, and it is accessible from outside the housing 50. The fluid-processing equipment 10 has an actuator 20 in the form of an electric motor that is connected directly to the shaft 62. The actuator 20 is stationary and fixed relative to the housing 50. This way, the actuator 20 is operationally coupled to the rotating element 48 and arranged to cause the rotating relative motion between the rotating element 48 and the housing 50.

The fluid-processing equipment 10 has a plurality of first magnets 16 located at and attached to the housing 50. This way, the first magnets 16 are supported by and fixed to the housing 50. The housing 50 has an inner side and an outer side, and the inner side faces the inner space 52. The first magnets 16 are located at the inner side and are fully embedded in the housing 50, and the housing 50 forms a first cover 28 that prevents the fluid from contacting the first magnets 16. This way, the first magnets 16 are isolated from the fluid. The first magnets 16 are spaced apart from the rotating element 48, and each of the first magnets 16 is positioned and oriented with one of its poles facing the impeller 70 of the rotating element 48.

The first magnets 16 are distributed along the rotational axis 32, and they are also distributed around the rotational axis 32 with a 180° separation between them, as shown in Fig. 1. In an alternative embodiment, there are additional first magnets 16 distributed around the rotational axis 32 but positioned with a 90° separation between them.

The centrifugal pump 66 has a rotary support 36 in the form of a rolling-element bearing that interconnects the shaft 62 and the housing 50. This way, the rotary support 36 operatively couples rotating element 48 and the housing 50, and in extension the rotating element 48 and the first magnets 16.

Each of the first magnets 16 is a permanent magnet in the form of neodymium-iron-boron magnets and thus arranged to generate a magnetic field. Each of the first magnets 16 are further arranged to subject a portion of the impeller 70 of the rotating element 48 to the magnetic field. The magnetic field of each magnet has a flux density between 1 mT and 10 mT at the impeller 70 of the rotating element 48.

Arranged as described above, the rotating element 48 constitutes a first element 12, and the housing 50 constitutes a second element 14. Each of the first magnets 16 subjects the first element 12 to a magnetic field and is arranged to form eddy currents in the first element 12 at the relative motion, and the eddy currents inhibit microbiological growth on the first element 12 at the relative motion.

The actuator 20 is arranged to operate the centrifugal pump 66 such that the rotating relative motion between the first magnets 16 and the rotating element 48 has a rotational rate below 2 500 rpm. The relative speed between the radially outermost first magnets 16 and impeller 70 of the rotating element 48 is less than 40 m/s at the first magnets 16. The electric motor of the actuator 20 consumes an average input power when causing the intended relative motion, and more than 8% of the average input power is converted to eddy currents by the first magnets 16.

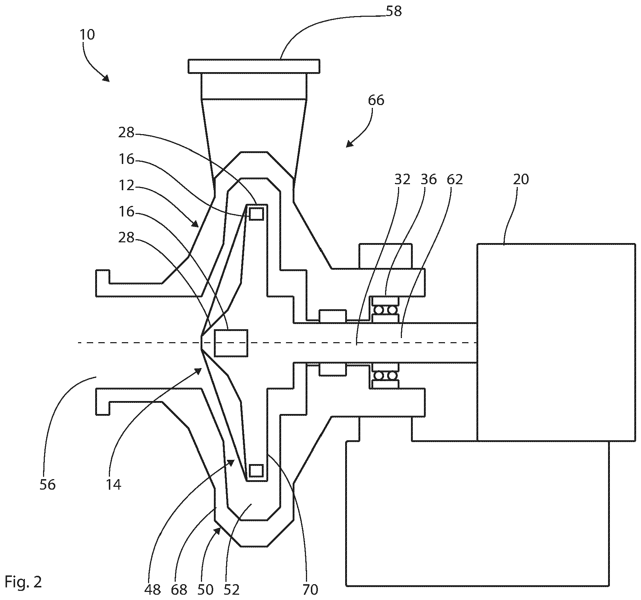

Fig. 2 shows another embodiment of a fluid-processing equipment 10 in the form of a centrifugal pump 66. It shares the features of the embodiment described in Fig. 1 but differs in that the first magnets 16 are located at and attached to impeller 70 of the rotating element 48. This means that the first magnets 16 are supported by and fixed to the rotating element 48. The first magnets 16 are fully embedded in the rotating element 48, and the impeller 70 of the rotating element 48 forms a first cover 28 that prevents the fluid from contacting the first magnets 16. This way, the first magnets 16 are isolated from the fluid. The first magnets 16 are spaced apart from the housing 50, and each of the radially outermost first magnets 16 is positioned and oriented with one of its poles facing radially outwards towards the housing 50. The central first magnet 16 is located at the rotational axis 32 and oriented with one of its poles facing the inlet 56.

The radially outermost first magnets 16 are distributed around the rotational axis 32 with a 180° separation between them, as shown in Fig. 1. In an alternative embodiment, there are additional first magnets 16, and the radially outermost are distributed around the rotational axis 32 with a 120° separation between them. The magnetic field of each magnet has a flux density between 1 mT and 10 mT at the housing 50.

Arranged as described above, the housing 50 constitutes a first element 12, and the rotating element 48 constitutes a second element 14. Each of the first magnets 16 each subjects the first element 12 to a magnetic field and is arranged to form eddy currents in the first element 12 at the relative motion, and the eddy currents inhibit microbiological growth on the first element 12 at the relative motion.

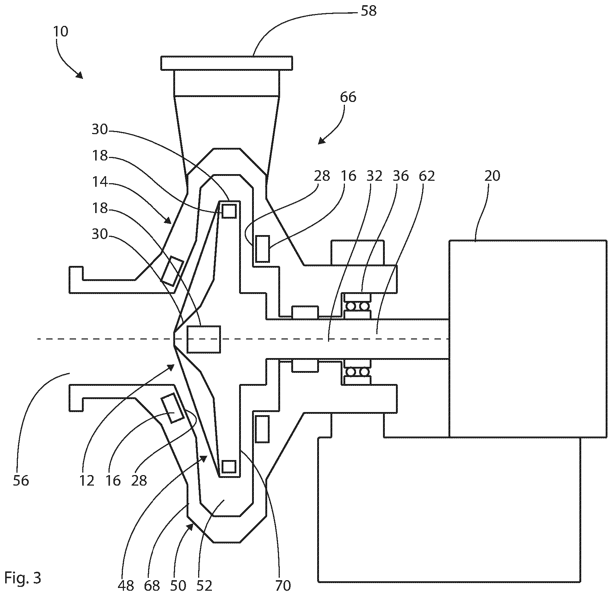

Fig. 3 shows another embodiment of a fluid-processing equipment 10 in the form of a centrifugal pump 66. It shares the features of the embodiment described in Fig. 1 but differs in that it further has a plurality of second magnets 18 located at and attached to the impeller 70 of the rotating element 48. The second magnets 18 are arranged as the first magnets 16 described in relation to Fig. 2. The second magnets 18 are embedded in the impeller 70. This way, the rotating element 48 forms a second cover 30 that covers the second magnets and isolates the second magnets from the fluid.

Arranged this way, the rotating element 48 constitutes a first element 12, and the housing 50 constitutes a second element 14. Each of the first magnets 16 subjects the first element 12 to a magnetic field and is arranged to form eddy currents in the first element 12 at the relative motion, and the eddy currents inhibit microbiological growth on the first element 12 at the relative motion. Each of the second magnets 18 subjects the second element 14 to a magnetic field and is arranged to form eddy currents in the second element 14 at the relative motion, and the eddy currents inhibit microbiological growth on the second element 14 at the relative motion.

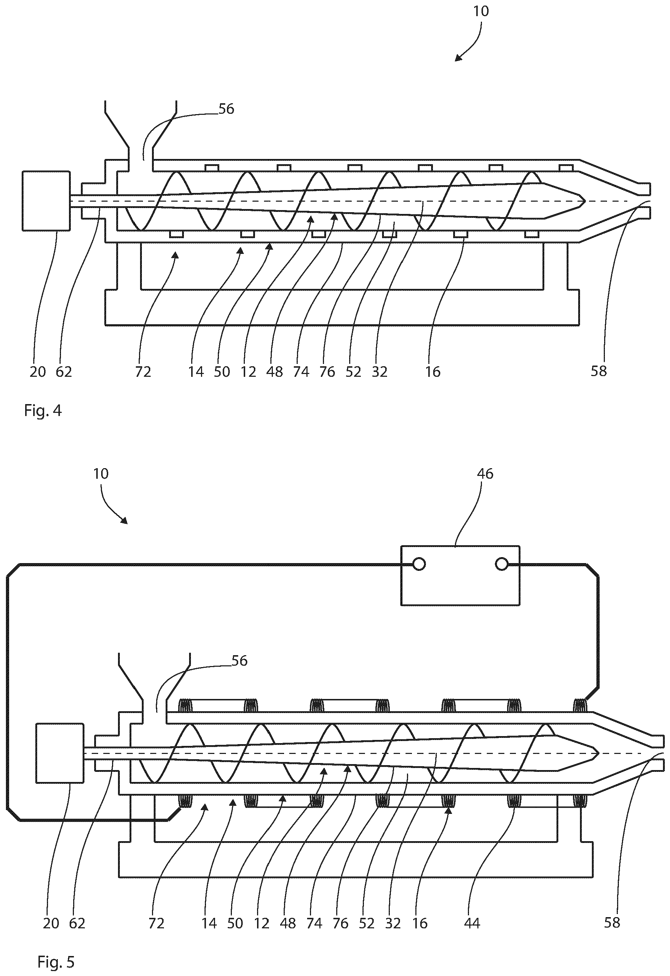

Fig. 4 shows an embodiment of a fluid-processing equipment 10 in the form of an extruder 72. The extruder 72 has a housing 50 and a rotating element 48. The housing 50 is static and the rotating element 48 is movable relative to the surroundings. The rotating element 48 rotates around a rotational axis 32 when operating the extruder 72. The housing 50 forms an inner space 52 for holding a fluid. This means that the housing 50 is configured to be exposed to the fluid. It further has an inlet 56 in the form of a hopper for receiving the fluid into the housing 50 and an outlet 58 in the form of a die for discharging the fluid from the housing 50. The inlet 56 is located off-axis relative to the rotational axis 32, and the outlet 58 is centred on the rotational axis 32.

The housing 50 has a casing 74 that that forms the inner space 52. The rotating element 48 has a rotor 76 and a shaft 62 that are centred on the rotational axis 32, and the rotor 76 is attached to the shaft 62. The rotor 76 is located within the casing 74, which means that it is configured to be exposed to the fluid within the housing 50. The rotor 76 is in the form of a screw and configured to generate a flow of the fluid from the inlet 56 to the outlet 58 at a rotating relative motion between the rotating element 48 and the housing 50.

The rotating element 48 is of a first material, and the first material is an austenitic stainless steel that is non-magnetic. The housing 50 is of a second material, and the second material is martensitic stainless and has a relative permeability that is less than 1000.

The shaft 62 extends from inside the housing 50 to outside the housing 50, and it is accessible from outside the housing 50. The fluid-processing equipment 10 has an actuator 20 in the form of an electric motor that is connected directly to the shaft 62. The actuator 20 is stationary and fixed relative to the housing 50. This way, the actuator 20 is operationally coupled to the rotating element 48 arranged to cause the rotating relative motion between the rotating element 48 and the housing 50.

The fluid-processing equipment 10 has a plurality of first magnets 16 located at and attached to the housing 50. Additionally, the first magnets 16 are supported by and fixed to the housing 50. The housing 50 has an inner side and an outer side, and the inner side faces the inner space 52. The first magnets 16 are located at the inner side and are fully embedded in the housing 50 and isolated from the fluid. The first magnets 16 are spaced apart from the rotating element 48, and each of the first magnets 16 is positioned and oriented with one of its poles facing the rotor 76 of the rotating element 48.

The first magnets 16 are distributed along the rotational axis 32. They are also distributed around the rotational axis 32 with a 180° separation between them, as shown in Fig. 4. In an alternative embodiment, the first magnets 16 has a different distributed around the rotational axis 32.

The extruder 72 has a rotary support (not shown) in the form of a rolling-element bearing that interconnects the shaft 62 and the housing 50. This way, the rotary support (not shown) operatively couples rotating element 48 and the housing 50, and in extension the rotating element 48 and the first magnets 16.

Each of the first magnets 16 is a permanent magnet in the form of neodymium-iron-boron magnets and thus arranged to generate a magnetic field. Each of the first magnets 16 are further arranged to subject a portion of the rotor 76 of the rotating element 48 to the magnetic field. The magnetic field of each magnet has a flux density between 10 mT and 100 mT at the rotor 76 of the rotating element 48.

Arranged as described above, the rotating element 48 constitutes a first element 12, and the housing 50 constitutes a second element 14. Each of the first magnets 16 subjects the first element 12 to a magnetic field and is arranged to form eddy currents in the first element 12 at the relative motion, and the eddy currents inhibit microbiological growth on the first element 12 at the relative motion.

The actuator 20 is arranged to operate the extruder 72 such that the rotating relative motion between the first magnets 16 and the rotating element 48 has a rotational rate below 150 rpm. The electric motor of the actuator 20 consumes an average input power when operating and causing the intended relative motion, and more than 1% of the average input power is converted to eddy currents by the first magnets 16.

Fig. 5 shows another embodiment of a fluid-processing equipment 10 in the form of an extruder 72. It shares the features of the embodiment described in Fig. 1 but differs in that the first magnets 16 are electromagnets in the form of coils 44, with each coil 44 encircling the housing 50, and in extension the rotating element 48. The fluid-processing equipment 10 further has a power source 46 arranged to supply each of the coils 44 with a current for forming the magnetic field of the first magnets 16. Each of the first magnets 16 are centered on the rotational axis 32 and oriented such that its magnetic axis is parallel with and coincides with the rotational axis 32. The first magnets 16 are distributed and spaced apart along the rotational axis 32.

The embodiment of Fig. 5 further differs in that the second material of the housing 50 is an austenitic stainless steel that is non-magnetic.

Arranged as described above, the rotating element 48 constitutes a first element 12, and the housing 50 constitutes a second element 14. Each of the first magnets 16 subjects the first element 12 to a magnetic field and is arranged to form eddy currents in the first element 12 at the relative motion and at a supply of current from the power source 46 to the first magnets 16, and the eddy currents inhibit microbiological growth on the first element 12 at the relative motion.

The electric motor of the actuator 20 consumes input power when operating and causing the intended relative motion, and more than 1% of the average input power is converted to eddy currents by the first magnets 16 at a supply of current from the power source 46 to the first magnets 16.

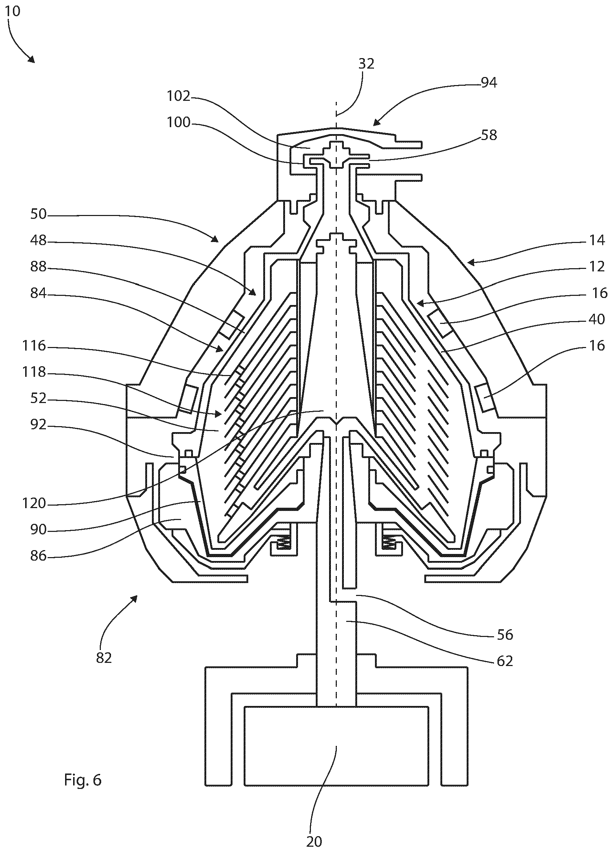

Fig. 6 shows an embodiment of a fluid-processing equipment 10 in the form of a centrifuge separator 82 for forming lighter and heavier phases of a fluid. The centrifuge separator 82 has a housing 50 and a rotating element 48. The housing 50 is static and the rotating element 48 is movable relative to the surroundings. The rotating element 48 rotates around a vertical rotational axis 32 when operating the centrifuge separator 82. The rotating element 48 has an inner space 52 for holding the fluid. This means that the rotating element 48 is configured to be exposed to the fluid. It further has an inlet 56 for receiving the fluid into the inner space 52 of the rotating element 48 and an outlet 58 for discharging the fluid from the inner space 52 of the rotating element 48. The inlet 56 and outlet 58 are located at the rotational axis 32 and at opposite ends of the rotating element 48.

The rotating element 48 has a bowl 84 that is located within the housing 50 and forms the inner space 52. The bowl 84 has a lower bowl body 86, an upper bowl body 88, and a sliding piston 90 that can slide relative to the lower bowl body 86 parallel to the rotational axis 32. These components are annular, centred on the rotational axis 32, and rotationally symmetric relative to the rotational axis 32.

The sliding piston 90 and the upper bowl jointly encloses the inner space 52. The sliding piston 90 has an upper position in which it seals to the upper bowl body 88 and a lower position in which the fluid, or sludge accumulated from the fluid, can be ejected from the inner space 52 between the upper bowl body 88 and the sliding piston 90. The lower bowl body 86 forms a plurality of ejection ports 92 through which fluid, or accumulated sludge, can pass after being ejected from the inner space 52.

The rotating element 48 further has a shaft 62 that is centred on the rotational axis 32, and the lower bowl body 86 of the bowl 84 is attached to the shaft 62. The shaft 62 extends from inside the housing 50 to outside the housing 50, and it is accessible from outside the housing 50. The inlet 56 is formed by the shaft 62, and it forms a fluid conduit that connects the inlet 56 to the inner space 52. The fluid-processing equipment 10 has an actuator 20 in the form of an electric motor that is coupled to the shaft 62. The actuator 20 is stationary and fixed relative to the housing 50. This way, the actuator 20 is operationally coupled to the rotating element 48 and arranged to cause a rotating relative motion between the rotating element 48 and the housing 50.

The rotating element 48 and the housing 50 jointly form a pump 94 in the form a centrifugal pump that can pump the fluid through the outlet 58 at the rotating relative motion. The pump 94 has a volute 102 that is formed by the housing 50 and an impeller 100 that forms part of the rotating element 48 and is located within the volute 102. This way, the pump 94 is arranged to pump the fluid through the outlet 58 at the rotating relative motion.

The centrifuge separator 82 is arranged to form a lighter phase of the fluid and a heavier phase of the fluid within the inner space 52 of the rotating element 48 at the rotating relative motion. The centrifuge separator 82 is arranged to discharge the lighter phase of the fluid through the outlet 58, and to discharge the heavier phase of the fluid through the ejection ports 92. The inlet 56 and the outlet 58 are located at opposite ends of the rotating element 48.

The rotating element 48 further has a plurality of discs 116 that are located in the in the inner space 52 and configured to enhance the formation of the lighter phase and the heavier phase of the fluid. This means that the discs 116 are exposed to the fluid. The discs 116 are annular, centred on the rotational axis 32 and arranged in a disc stack 118 that is aligned with the rotating axis. Each disc 116 outlines a truncated cone with a narrow end facing upward and a wide end facing downward. Each pair of neighbouring discs 116 form an intermediate space between them in which the fluid can flow at the rotating relative motion.

The rotating element 48 further has a distributer 120 that is located in the inner space 52 and configured to distribute a flow of the fluid received via the inlet 56 within the inner space 52. This means that the distributer 120 is exposed to the fluid. The distributer 120 is rotationally locked to the lower bowl body 86 and configured to lead a flow of the fluid to the disc stack 118. Each disc 116 has a central aperture and the distributer 120 has a core that is centred on the rotational axis 32, extends through the aperture of each disc, and aligns the disc stack 118 with the rotational axis 32. The core forms a plurality of radial fins that extend along the rotational axis 32, and each radial fin contacts the discs. This way, the distributer 120 supports the disc stack 118. Each pair of radial fins forms a gap between them that allows passage of the fluid from the disc stack 118 towards to the outlet 58.

The components of the rotating element 48 are of non-magnetic austenitic stainless steel, and the second material is of cast iron.

The fluid-processing equipment 10 has a plurality of first magnets 16 located at and attached to the housing 50. This means that the first magnets 16 are supported by and fixed to the housing 50. The housing 50 has an inner side and an outer side, and the inner side faces the rotating element 48. The first magnets 16 are located at the inner side of the housing 50 and are isolated from the fluid within the rotating element 48. The first magnets 16 are spaced apart from the rotating element 48, and each of the first magnets 16 is positioned and oriented with one of its poles facing the rotating element 48.

Each of the first magnets 16 are further arranged to subject a portion of the bowl 84 of the rotating element 48 to the magnetic field. The magnetic field of each magnet has a flux density between 0.5 mT and 1 mT at the bowl 84 of the rotating element 48.

There are sixteen first magnets 16 divided in two groups of eight first magnets 16. The two groups are spaced apart along the rotational axis 32, and the first magnets 16 within each group are arranged in a circle centred on the rotational axis 32. The first magnets 16 are positioned pairwise with opposite polarities facing the rotating element 48 so that the magnetic fields of each pair of first magnets 16 are aligned at the rotating element 48. Each of the first magnets 16 is a permanent magnet in the form of neodymium-iron-boron magnets and thus arranged to generate a magnetic field. The first magnets 16 are located at the housing 50, and the housing 50 is of cast iron. This way, the housing 50 is arranged to lead the magnetic field between the first magnets 16 of each pair. The pairs of first magnets 16 are distributed around the rotational axis 32 with a 90° separation between them.

The bowl 84, or more precisely the upper bowl body 88, of the rotating element 48 has a first wall 40 that is configured to be exposed to the fluid and located between the first magnets 16 and the fluid. The first magnets 16, or more precisely the pairs of first magnets 16, are arranged to form eddy currents in the first wall 40 at the relative motion. The magnetic fields from the pairs of first magnets 16 pass partly through the first wall 40 at the relative motion and reach a portion of the disc stack 118.

Arranged as described above, the rotating element 48 constitutes a first element 12, and the housing 50 constitutes a second element 14. Each of the first magnets 16 subjects the first element 12 to a magnetic field and is arranged to form eddy currents in the first element 12, or more precisely in the first wall 40 and the abovementioned portions of the disc stack 118, at the relative motion, and the eddy currents inhibit microbiological growth on the first element 12 at the relative motion, or more precisely on the mentioned parts of the rotating element 48.

The actuator 20 is arranged to operate the centrifuge separator 82 such that the rotating relative motion between the first magnets 16 and the rotating element 48 has a rotational rate below 10 000 rpm. The relative speed between the radially outermost first magnets 16 and the rotating element 48 is less than 160 m/s at the first magnets 16. The electric motor of the actuator 20 consumes an average input power when causing the intended relative motion, and more than 8% of the average input power is converted to eddy currents by the first magnets 16.

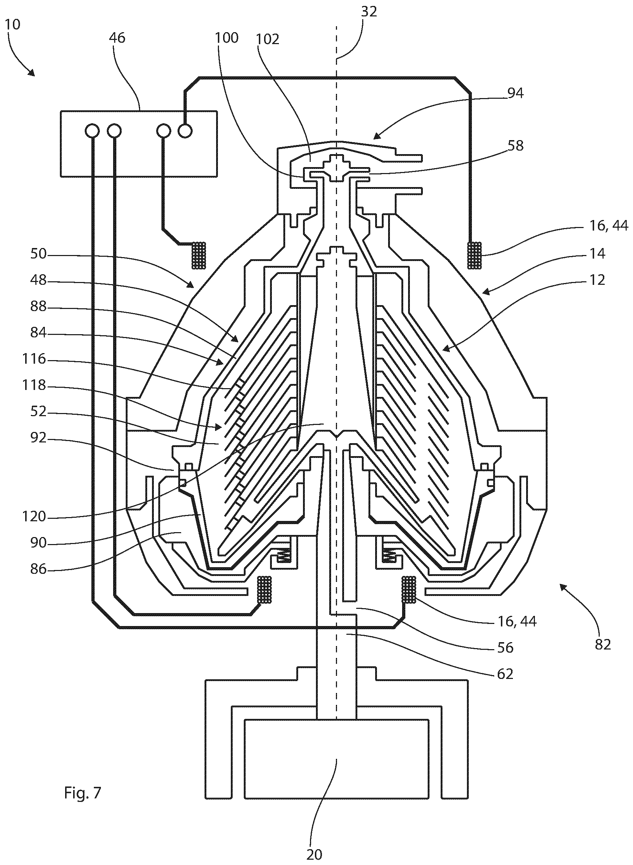

Fig. 7 shows another embodiment of a fluid-processing equipment 10 in the form of a centrifuge separator 82. It shares the features of the embodiment described in Fig. 6 but differs in that the first magnets 16 are electromagnets in the form of a pair of coils 44. One of the coils 44 encircles the housing 50 and the bowl 84, and the other coil 44 encircles the shaft 62. In extension, the coils 44 encircle the rotating element 48. The fluid-processing equipment 10 further has a power source 46 arranged to supply each of the coils 44 with a current for forming the magnetic field. The current is set such that the magnetic field of each first magnet 16 is greater than 1 mT at the bowl 84 of the rotating element 48. Each of the first magnets 16 are centered on the rotational axis 32 and oriented such that its magnetic axis is parallel with and coincides with the rotational axis 32. The first magnets 16 are distributed and spaced apart along the rotational axis 32. In an alternative embodiment, the coils 44 are located between the housing 50 and the rotating element 48.

The embodiment of Fig. 7 further differs in that the second material of the housing 50 is an austenitic stainless steel that is non-magnetic. Arranged as shown in Fig. 7, the combined first magnets 16 subjects the complete rotating element 48, including the bowl 84, disc stack 118, distributer 120, and impeller 100, to a magnetic field. This way, the rotating element 48 constitutes a first element 12, and the housing 50 constitutes a second element 14. Each of the first magnets 16 subjects the first element 12 to a magnetic field and is arranged to form eddy currents in the first element 12 at the relative motion and at a supply of current from the power source 46 to the first magnets 16, and the eddy currents inhibit microbiological growth on the first element 12 at the relative motion.

The electric motor of the actuator 20 consumes input power when operating and causing the intended relative motion, and more than 2% of the average input power is converted to eddy currents by the first magnets 16 at a supply of current from the power source 46 to the first magnets 16.

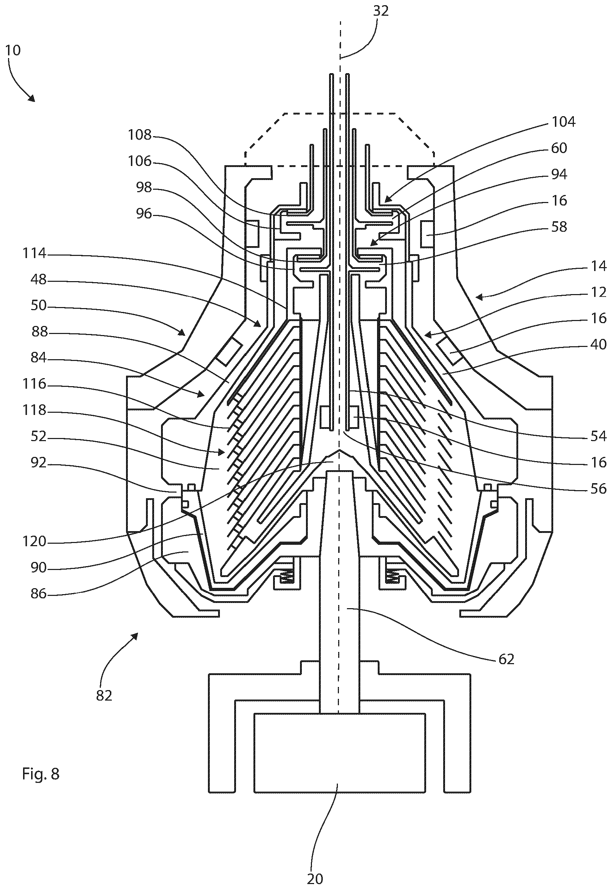

Fig. 8 shows another embodiment of a fluid-processing equipment 10 in the form of a centrifuge separator 82. It shares the features of the embodiment described in Fig. 6 but differs in that the rotating element 48 further has an additional outlet 60 for discharging the fluid from the inner space 52 of the rotating element 48. The outlet 58 is configured to discharge a lighter phase of the fluid, and the additional outlet 60 is configured to discharge a heavier phase of the fluid. The rotating element 48 further has a partition 114 that is located in the inner space 52 of the rotating element 48 arranged to guide the lighter phase of the fluid the outlet 58 and the heavier phase to the additional outlet 60. The partition 114 is rotationally locked to the upper bowl body 88 of the bowl 84. The partition 114 is annular and centred on the rotational axis 32. It is located at the upper bowl body 88 and arranged to allow the heavier phase to pass between the partition 114 and the upper bowl body 88.

Instead of a centrifugal pump 66, the rotating element 48 and the housing 50 jointly form a pump 94 in the form a centripetal pump that can pump the fluid through the outlet 58 at the rotating relative motion. The centripetal pump 94 has a paring chamber 96 that is formed by the rotating element 48 and a paring disc 98 that forms part of the housing 50 and is located within the paring chamber 96. This way, the pump 94 is arranged to pump the lighter phase of the fluid through the outlet 58 at the rotating relative motion.

Further, the rotating element 48 and the housing 50 jointly form an additional pump 104 in the form an additional centripetal pump that can pump the fluid through the additional outlet 60 at the rotating relative motion. The additional centripetal pump has an additional paring chamber 106 that is formed by the rotating element 48 and an additional paring disc 108 that forms part of the housing 50 and is located within the additional paring chamber 106. This way, the additional pump 104 is arranged to pump the heavier phase of the fluid through the additional outlet 60 at the rotating relative motion.

Further, the centrifuge separator 82 has an inlet pipe 54 that is static relative to the surroundings and extends from outside the rotating element 48 into the inner space 52 of the bowl 84. The inlet pipe 54 is centred on and extends along the rotating axis and enters inner space 52 from above via the upper bowl body 88. The inlet pipe 54 has an outer open end that is located outside the rotating element 48 for receiving the fluid and an inner open end that is located within the rotating element 48. This way, the inlet 56 of the rotating element 48 is located within the bowl 84 and the inner space 52, and the inlet pipe 54 constitutes an inner part 54 that extends into and is partly located within the inner space 63 of the rotating element 48.

The inlet pipe 54 is of ferritic stainless steel and the components of the pump and the additional pump 104 are of non-magnetic austenitic stainless steel, the remining components of the rotating element 48 are of non-magnetic austenitic stainless steel, and the remaining parts of the housing 50 are of cast iron.

As in the embodiment of Fig. 6, the fluid-processing equipment 10 has a plurality of first magnets 16 that are permanent magnets in the form of neodymium-iron-boron magnets. The first magnets 16 are divided into two sets. The first set is located at and attached to the housing 50, and the second set is located at and attached to the inlet pipe 54. This means that the first magnets 16 of the first set are located outside the rotating element 48, and the first magnets 16 of the second set are located within the rotating element 48, or more precisely in the inner space 52. This means that the first magnets 16 of the first set are supported by and fixed to the housing 50, and the first magnets 16 of the second set are supported by and fixed to the inlet pipe 54.

There are sixteen first magnets 16 in the first set, which is divided in two groups of eight first magnets 16. The two groups are spaced apart along the rotational axis 32, and the first magnets 16 within each group are arranged in a circle centred on the rotational axis 32. The first magnets 16 of the first set are positioned pairwise with opposite polarities facing the rotating element 48 so that the magnetic fields of each pair of first magnets 16 are aligned at the rotating element 48. The first magnets 16 of the first set are located at the housing 50, and the housing 50 is of cast iron. This way, the housing 50 is arranged to lead the magnetic field between the first magnets 16 of each pair. The pairs of first magnets 16 are distributed around the rotational axis 32 with a 90° separation between them.

There are two magnets of the second set. They are positioned on opposite sides of the inlet pipe 54 and oriented with the poles aligned such that the magnetic fields combine via the inlet pipe 54 of ferritic stainless steel. The first magnets 16 of the second set are positioned with a 180° separation between them relative to the rotational axis 32.

The housing 50 has an inner side and an outer side, and the inner side faces the rotating element 48. The first magnets 16 of the first set are located at the inner side of the housing 50 and are isolated from the fluid within the rotating element 48. The firs magnets of the second set are located in the inner space 52 and exposed to the fluid within the rotating element 48. The first magnets 16 are spaced apart from the rotating element 48, and each of the first magnets 16 is positioned and oriented with one of its poles facing the rotating element 48.

Each of the first magnets 16 are further arranged to subject a portion of the bowl 84 of the rotating element 48 to the magnetic field. The magnetic field of each of the first magnets 16 of the first set has a flux density between 0.5 mT and 1 mT at the bowl 84 of the rotating element 48, and the magnetic field of each of the first magnets 16 of the second set has a flux density between 1 mT and 10 mT at distributer 120 of the rotating element 48.

The bowl 84, or more precisely the upper bowl body 88, of the rotating element 48 has a first wall 40 that is configured to be exposed to the fluid and located between the first magnets 16 of the first set and the fluid. The first magnets 16, or more precisely the pairs of first magnets 16, of the first set, are arranged to form eddy currents in the first wall 40 at the relative motion. The magnetic fields from the first magnets 16 of one of the groups of the first set pass partly through the first wall 40 at the relative motion between and reach a portion of the disc stack 118, and the magnetic fields from the first magnets 16 of the other group of the first set pass partly through the first wall 40 at the relative motion between and reach the paring chamber 96 and the additional paring chamber 106. The magnetic fields from the first magnets 16 of the second set pass partly through the distributer 120 at the relative motion between and reach a portion of the disc stack 118.

Arranged as described above, the rotating element 48 constitutes a first element 12, and the housing 50 and the inlet pipe 54 jointly constitute a second element 14. Each of the first magnets 16 subjects the first element 12 to a magnetic field and are arranged to form eddy currents in the first element 12, or more precisely in the first wall 40, the partition 114, the disc stack 118, the distributer 120, the paring chamber 96, and the additional paring chamber 106 at the relative motion, and the eddy currents inhibit microbiological growth on these components of the first element 12 at the relative motion, or more precisely on the mentioned parts of the rotating element 48.

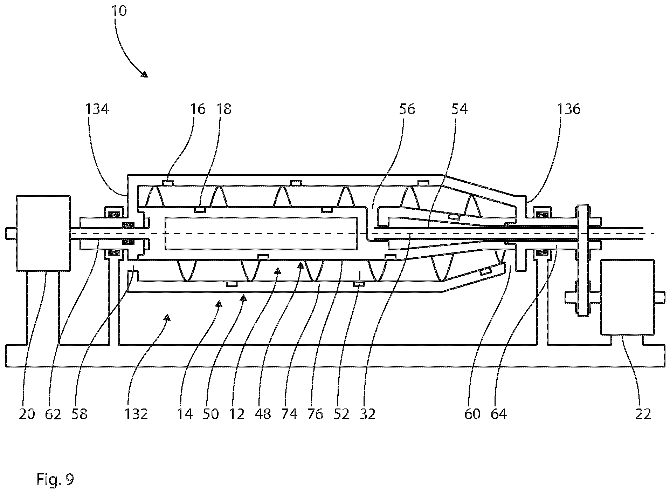

Fig. 9 shows an embodiment of a fluid-processing equipment 10 in the form of a decanter centrifuge 132. The decanter centrifuge 132 has a rotating housing 50 and a rotating element 48. The rotating housing 50 and the rotating element 48 are movable relative to the surroundings and can rotate around a common rotational axis 32 when operating the decanter centrifuge 132. The rotating housing 50 and the rotating element 48 are arranged to rotate in the same direction relative to the rotational axis 32, but with the rotating housing 50 rotating at a higher rate than the rotating element 48.

The rotating housing 50 forms an inner space 52 for holding a fluid. This means that the rotating housing 50 is configured to be exposed to the fluid.

The rotating element 48 has an inlet 56 for introducing the fluid into the rotating housing 50. The decanter centrifuge 132 has an inlet pipe 54 that extends from outside the rotating housing 50 into the rotating element 48, and in extension into the inner space 52 of the rotating housing 50. The fluid is introduced into the rotating inner space 52 and the rotating housing 50 via the inlet pipe 54. The inlet pipe 54 is static relative to the surroundings. The inlet pipe 54 is centred on and extends along the rotating axis. It has an outer open end that is located outside the rotating element 48 for receiving the fluid and an inner open end that is located within the rotating element 48 and in fluid communication with the inlet 56 of the rotating element 48.

The rotating housing 50 has an outlet 58 and an additional outlet 60, and the inlet 56 is located between the outlet 58 and the additional outlet 60. The rotating housing 50 has a first end 134 and an opposite second end 136 along the rotational axis 32. The outlet 58 is located at the first end 134 and the additional outlet 60 is located at the second end 136.

The rotating element 48 has a rotor 76 in the form of a scroll. The rotor 76 is located within the casing 74, which means that it is configured to be exposed to the fluid within the rotating housing 50. The rotating element 48 further has a rotating-element shaft 62 that is located at the first end 134 of the rotating housing 50, and the rotor 76 is attached to the rotating-element shaft 62.

The rotating housing 50 has a casing 74 that forms the inner space 52. The inner space 52 of the rotating housing 50, or more precisely the casing 74, outlines a cylinder at the first end 134, and it tapers towards the rotational axis at the second end 136. The rotating housing 50 further has a rotating-housing shaft 64 that is located at the second end 136 of the rotating housing 50, and the casing 74 is attached to the rotating-hosing shaft 62. The inlet pipe 54 extends through the rotating-housing shaft 64. The rotating-element shaft 62 and the rotating-housing shaft 64 are both centred on the rotational axis 32.

The rotating-element shaft 62 extends from inside the rotating housing 50 to outside the rotating housing 50 and is accessible from outside the rotating housing 50. The fluid-processing equipment 10 has a first actuator 20 in the form of an electric motor that is connected to the rotating-element shaft 62. The fluid-processing equipment 10 further has a second actuator 22 in the form of an electric motor that is connected to the rotating-housing shaft 64. The first actuator 20 and the second actuator 20 are stationary and fixed relative to the surroundings. This way, the first actuator 20 and the second actuator 20 are operationally coupled to the rotating element 48 and arranged to cause a rotating relative motion between the rotating element 48 and the rotating housing 50.

Arranged this way, the rotating housing 50 can form a lighter phase and a heavier phase of the fluid in the inner space 52 at the rotating relative motion, and the rotor 76 can generate a flow of the heavier phase of the fluid towards the second end 136 of the rotating housing 50 and the additional outlet 60 at the rotating relative motion. The lighter phase is discharged via the outlet 58 at the first end 134 of the rotating housing 50.

The rotating element 48 is of a first material, and the first material is an austenitic stainless steel that is non-magnetic. The rotating housing 50 is of a second material, and the second material is an austenitic stainless steel that is non-magnetic.

The fluid-processing equipment 10 has a plurality of first magnets 16 located at and attached to the casing 74 of the rotating housing 50. This means that the first magnets 16 are supported by and fixed to the rotating housing 50. The rotating housing 50 has an inner side and an outer side, and the inner side faces the inner space 52. The first magnets 16 are located at the inner side and are fully embedded in the rotating housing 50 and isolated from the fluid. The first magnets 16 are spaced apart from the rotating element 48, and each of the first magnets 16 is positioned and oriented with one of its poles facing the rotor 76 of the rotating element 48. The first magnets 16 are distributed along the rotational axis 32. They are also distributed around the rotational axis 32 with a 180° separation between them.

The fluid-processing equipment 10 further has a plurality of second magnets 18 located at and attached to the rotor 76 of the rotating element 48. Additionally, the second magnets 18 are supported by and fixed to the rotating element 48. The second magnets 18 are spaced apart from the rotating housing 50, and each of the second magnets 18 is positioned and oriented with one of its poles facing the casing 74 of the rotating housing 50. The second magnets 18 are distributed along the rotational axis 32. They are also distributed around the rotational axis 32 with a 180° separation between them.

Each of the first magnets 16 and the second magnets 18 is a permanent magnet in the form of neodymium-iron-boron magnets and thus arranged to generate a magnetic field. Each of the first magnets 16 are further arranged to subject a portion of the rotor 76 of the rotating element 48 to the magnetic field, and each of the second magnets 18 are further arranged to subject a portion of the basing 74 of the rotating housing 50 to the magnetic field. The magnetic field of each of the first magnets 16 has a flux density between 10 mT and 100 mT at the rotor 76 of the rotating element 48, and the magnetic field of each of the second magnets 18 has a flux density between 10 mT and 100 mT at the casing 74 of the rotating housing 50.

Arranged as described above, the rotating element 48 constitutes a first element 12, and the rotating housing 50 constitutes a second element 14. Each of the first magnets 16 are arranged to form eddy currents in the first element 12 at the relative motion that inhibit microbiological growth on the first element 12 at the relative motion. Further, each of the second magnets 18 are arranged to form eddy currents in the second element 14 at the relative motion that inhibit microbiological growth on the second element 14.

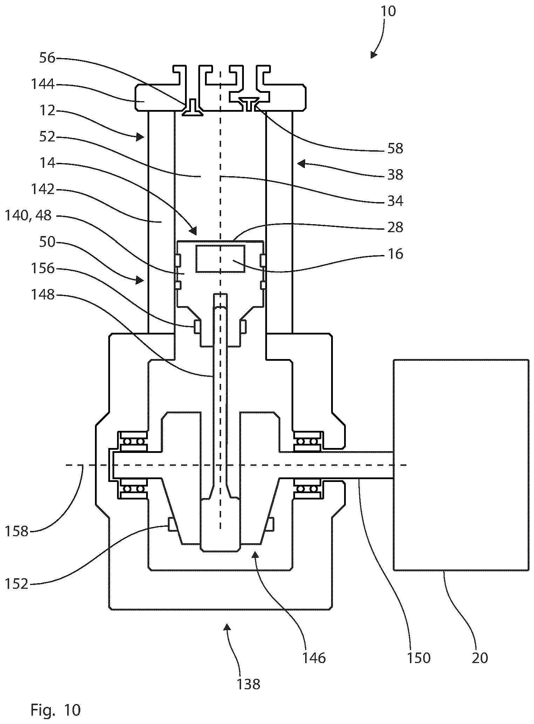

Fig. 10 shows an embodiment of a fluid-processing equipment 10 in the form of a positive-displacement pump for pumping a fluid, or more precisely a liquid. The positive-displacement pump is a piston pump 138 and has a housing 50 and a reciprocating element 48. The housing 50 is static and the reciprocating element 48 is movable relative to the surroundings. The reciprocating element 48 is a piston 140 that can reciprocate along a displacement axis 34 in a linear relative motion between the reciprocating element 48 and the housing 50 when operating the piston pump 138. Both the housing 50 and the piston 140 are of non-magnetic austenitic stainless steel.

The housing 50 has an inner space 52 for holding the fluid. This means that the housing 50 is configured to be exposed to the fluid. It further has an inlet 56 for receiving the fluid into the housing 50 and an outlet 58 for discharging the fluid from the housing 50. The inlet 56 and the outlet 58 are located at the same side of the housing 50.

The housing 50 has a cylinder block 142 that forms a cylinder in which the piston 140 is located and can reciprocate. This means that the piston 140, or more precisely the reciprocating element 48, is configured to be exposed to the fluid. The cylinder block 142 forms a linear support 38 that slidably supports the piston 140 relative to the housing 50. The housing 50 further has a cylinder head 144 that is attached and sealed to the cylinder block 142, and the cylinder block 142 and the cylinder head 144 jointly form the inner space 52. The cylinder head 144 forms the inlet 56 and the outlet 58. This way, the piston 140 is configured to generate a flow of the fluid into the inner space 52 via the inlet 56 and from the inner space 52 via the outlet 58 at the linear relative motion of the piston 140.

The piston pump 138 further has a shaft arrangement 146. The shaft arrangement 146 has a connecting rod 148 and a crankshaft 150, and the crankshaft 150 is connected to the piston 140 via the connecting rod 148. The crankshaft 150 forms a crank pin 152, the shaft arrangement 146 has a rod bearing (not shown) in the form of a rolling element bearing, and the connecting rod 148 is connected to the crank pin 152 via the rod bearing (not shown). The shaft arrangement 146 further has a piston pin 156 that pivotally connects the connecting rod 148 to the piston 140. The crankshaft 150 has a crankshaft axis 158 that is perpendicular to the displacement axis 34 and around which the crankshaft 150 can rotate. This way, the crankshaft 150 and the connecting rod 148 are arranged to convert a rotation of the crankshaft 150 to the reciprocating linear relative motion between the piston 140 and the housing 50.

The fluid-processing equipment 10 has an actuator 20 in the form of an electric motor that is connected directly to the crankshaft 150. The actuator 20 is stationary and fixed relative to the housing 50. This way, the actuator 20 is operationally coupled to the reciprocating element 48 and arranged to cause the linear relative motion between the reciprocating element 48 and the housing 50.

The fluid-processing equipment 10 has a single first magnet 16 located at and attached to the reciprocating element 48, or more precisely the piston 140. Additionally, the first magnet 16 is supported by and fixed to the piston 140. The first magnet 16 is fully embedded in the piston 140, and the piston 140 forms a first cover 28 that prevents the fluid from contacting the first magnet. This way, the first magnet 16 is isolated from the fluid. The first magnet 16 is spaced apart from the housing 50 and oriented with one of its poles facing the cylinder head 144.

The linear support 38 formed by the cylinder bloc of the housing 50 operatively couples reciprocating element 48 and the housing 50, and in extension the first magnet 16 and the housing 50. The first magnet 16 is a permanent magnet in the form of neodymium-iron-boron magnet, and it is arranged to subject the cylinder block 142 and the cylinder head 144 of the housing 50 to a magnetic field. The magnetic field has a flux density between 1 mT and 10 mT at the housing 50.

Arranged as described above, the housing 50 constitutes a first element 12, and the reciprocating element 48, or more precisely the piston 140, constitutes a second element 14. The first magnet 16 subjects the first element 12 to a magnetic field and is arranged to form eddy currents in the first element 12 at the relative motion, and the eddy currents inhibit microbiological growth on the first element 12 at the relative motion.

The actuator 20 is arranged to operate the piston pump 138 such that the linear relative motion has a cycle rate below 60 complete strokes per minute and above 30 complete strokes per minute, and the relative mean speed between the reciprocating element 48 and the housing 50, and in extension between the first magnet 16 and the housing 50, is between 0.5 m/s and 1 m/s. The electric motor of the actuator 20 consumes an average input power when operating, and more than 2% of the average input power is converted to eddy currents by the first magnet.

### Item list

- 10: fluid-processing equipment
- 12: first element
- 14: second element
- 16: first magnet
- 18: second magnet
- 20: actuator or first actuator
- 22: second actuator
- 28: first cover
- 30: second cover
- 32: rotational axis
- 34: displacement axis
- 36: rotary support
- 38: linear support
- 40: first wall
- 44: coil
- 46: power source
- 48: rotating element or reciprocating element
- 50: housing or rotating housing
- 52: inner space
- 54: inner part or inlet pipe
- 56: inlet
- 58: outlet
- 60: additional outlet
- 62: shaft or rotating-element shaft
- 64: rotating-housing shaft
- 66: centrifugal pump
- 68: volute casing
- 70: impeller
- 72: extruder
- 74: casing
- 76: rotor
- 82: centrifuge separator
- 84: bowl
- 86: lower bowl body
- 88: upper bowl body
- 90: sliding piston
- 92: ejection ports
- 94: pump
- 96: paring chamber
- 98: paring disc
- 100: impeller
- 102: volute
- 104: additional pump
- 106: additional paring chamber
- 108: additional paring disc
- 114: partition
- 116: disc
- 118: disc stack
- 120: distributer
- 132: decanter centrifuge
- 134: first end
- 136: second end
- 138: piston pump
- 140: piston
- 142: cylinder block
- 144: cylinder head
- 146: shaft arrangement
- 148: connecting rod
- 150: crankshaft
- 152: crank pin
- *154*: *rod bearing*
- 156: piston pin
- 158: crankshaft axis

## Claims

1. A fluid-processing equipment (10) for processing a fluid susceptible to microbiological growth, wherein the equipment (10) comprises:
- a first element (12),
- an actuator (20), and
- one or more first magnets (16), wherein
the first element (12) is configured to be exposed to the fluid,
the actuator (20) is arranged to cause a relative motion between the first element (12) and the first magnets (16), and
each of the first magnets (16) is arranged to form eddy currents in the first element (12) that inhibits microbiological growth on the first element (12) at the relative motion.

2. The fluid-processing equipment (10) according to claim 1, wherein the equipment (10) further comprises:
- a second element (14), wherein
the one or more first magnets (16) are supported by the second element (14).

3. The fluid-processing equipment (10) according to claim 2, wherein the actuator (20) is an electric motor, the actuator (20) is arranged to operate the equipment (10), the electric motor causes the relative motion at an average input power to the electric motor, and more than 1%, 2%, or 4% of the average input power is converted to eddy currents by the first magnets (16).

4. The fluid-processing equipment (10) according to claim 2 or 3, wherein the first magnets (16) are permanent magnets.

5. The fluid-processing equipment (10) according to claim 2 or 3, wherein the first magnets (16) are electromagnets, each electromagnet comprise a coil (44) arranged to generate a magnetic field at a current through the coil (44), and the equipment (10) further comprises:
- a power source (46), wherein
the power source (46) is arranged to supply each of the coils (44) with a current.

6. The fluid-processing equipment (10) according to any of the claims 2 to 5, wherein the equipment (10) further comprises:
- one or more second magnets (18), wherein
the second element (14) is configured to be exposed to the fluid, each of the second magnets (18) are arranged to form eddy currents in the second element (14) that inhibits microbiological growth on the second element (14) at the relative motion.

7. The fluid-processing equipment (10) according to claim 6, wherein the one or more second magnets (18) are supported by the first element (12).

8. The fluid-processing equipment (10) according to any of the claims 2 to 7, wherein the first element (12) is electrically conductive and non-magnetic, and the second element (14) is electrically conductive and non-magnetic.

9. The fluid-processing equipment (10) according to any of the claims 2 to 8, wherein the relative motion is a rotating relative motion.

10. The fluid-processing equipment (10) according to claim 9, wherein the second element (14) is static, and the first element (12) is movable.

11. The fluid-processing equipment (10) according to claim 10, wherein the first element (12) is a rotating element (48), and the second element (14) is a housing (50).

12. The fluid-processing equipment (10) according to claim 9, wherein the first element (12) is static, and the second element (14) is movable.

13. The fluid-processing equipment (10) according to claim 12, wherein the first element (12) is a housing (50), and the second element (14) is a rotating element (48).

14. The fluid-processing equipment (10) according to claim 9, wherein the first element (12) is movable, and the second element (14) is movable.

15. The fluid-processing equipment (10) according to any of the claims 2 to 8, wherein the relative motion is a linear relative motion.
